# FASCICULE DE BREVET EUROPEEN

(11) **EP 0 946 580 B1**
(45) Date de publication et mention de la délivrance du brevet: **03.04.2002**
(21) Numéro de dépôt: 97952973.2
(22) Date de dépôt: 22.12.1997
(51) Int. Cl.: C07H 17/08, A61K 31/70

(54) **NOUVEAUX DERIVES DE L'ERYTHROMYCINE, LEUR PROCEDE DE PREPARATION ET LEUR APPLICATION COMME MEDICAMENTS**
ERYTHROMYCIN-DERIVATE, VERFAHREN ZU IHRER HERSTELLUNG UND IHRER VERWENDUNG
NOVEL ERYTHROMYCIN DERIVATIVES, METHOD OF PREPARATION AND APPLICATION AS MEDICINES

(30) Priorité: 23.12.1996 FR 9615830
(43) Date de publication de la demande: 06.10.1999
(73) Titulaire: Aventis Pharma S.A., 92160 Antony (FR)
(72) Inventeur: AGOURIDAS, Constantin, F-94130 Nogent sur Marne (FR); CHANTOT, Jean-François, F-94130 Nogent sur Marne (FR)
(86) Numéro de dépôt international: FR9702380
(87) Numéro de publication internationale: WO9828316

(56) Documents cités:
- EP-A- 0 487 411

## Description

La présente invention concerne de nouveaux dérivés de l'érythromycine, leur procédé de préparation et leur application comme médicaments.

L'invention a pour objet les composés de formule (I) : 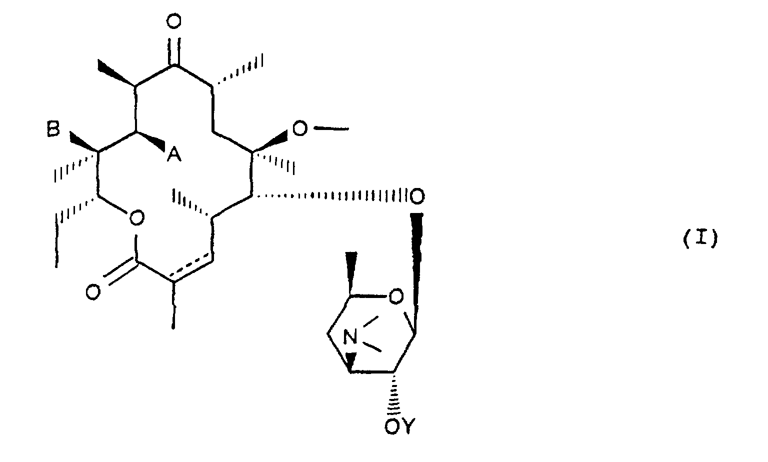 dans lesquels :
- ou bien A et B représentent un radical OH,
- ou bien B représente un radical OH et A forme avec le carbone qui le porte et le carbone en 10 une double liaison,
- ou bien A et B forment ensemble un radical carbonate,
- ou bien A et B forment ensemble avec les atomes de carbone qui les portent un cycle : 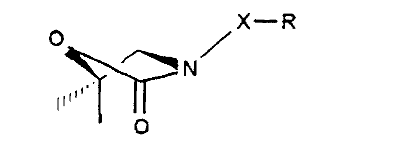
dans lesquels X représente un groupement CH₂, NH ou SO₂, R représente un radical (CH₂)ₙAr, N-(CH₂)ₙAr ou N=CH(CH₂)ₙAr, dans lequel n représente un nombre entier compris entre 1 et 6 et Ar représente un radical aryle ou hétéroaryle, éventuellement substitué, les traits pointillés représentant une double liaison éventuelle en 2(3), et Y représente un atome d'hydrogène ou le reste d'un acide organique carboxylique renfermant jusqu'à 18 atomes de carbone ainsi que leurs sels d'addition avec les acides.

Le radical aryle peut être un radical phényle ou naphtyle.

Le radical aryle peut être également un radical hétérocyclique substitué ou non comme le radical thiényle, furyle, pyrolyle, thiazolyle, oxazolyle, imidazolyle, thiadiazolyle, pyrazolyle ou isopyrazolyle, un radical pyridyle, pyrimidyle, pyridazinyle ou pyrazinyle, ou encore un radical indolyle benzofurannyle, benzothiazyle ou quinoléinyle.

Ces radicaux aryles peuvent comporter un ou plusieurs groupements choisis dans le groupe constitué par les radicaux hydroxyle, les atomes d'halogène, les radicaux NO₂, NH₂, C≡N, les radicaux alkyle, alkényle ou alkynyle, O-alkyle, O-alkényle ou O-alkynyle, S-alkyle, S-alkényle ou S-alkynyle et N-alkyle, N-alkényle ou N-alkynyle, renfermant jusqu'à 12 atomes de carbone éventuellement substitués par un ou plusieurs atomes d'halogène, le radical 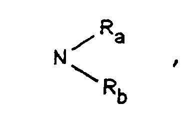 Ra et Rb identiques ou différents, représentant un atome d'hydrogène ou un radical alkyle renfermant jusqu'à 12 atomes de carbone, le radical 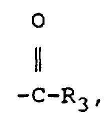 R₃ représentant un radical alkyle renfermant jusqu'à 12 atomes de carbone, ou un radical aryle ou hétéroaryle éventuellement substitué, les radicaux aryle, 0-aryle ou S-aryle carboxyliques ou aryle, O-aryle ou S-aryle hétérocycliques à 5 ou 6 chaînons comportant un ou plusieurs hétéroatomes, éventuellement substitués par un ou plusieurs des substituants mentionnés ci-dessous.
Comme hétérocycle préféré, on peut citer entre autres 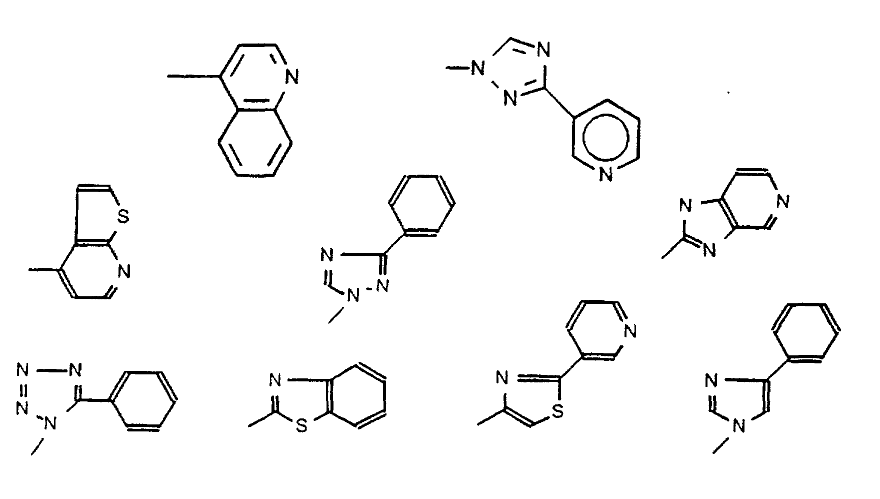 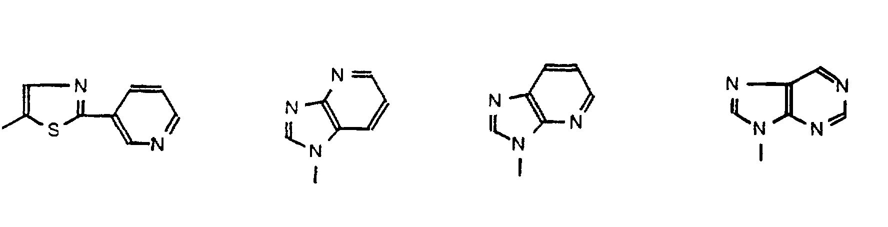 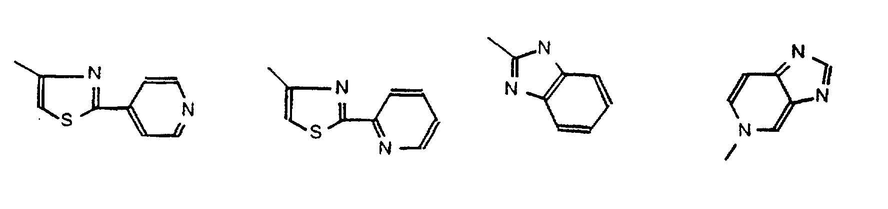 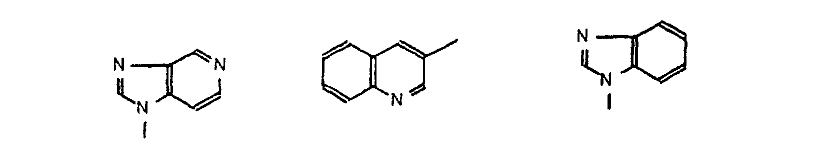 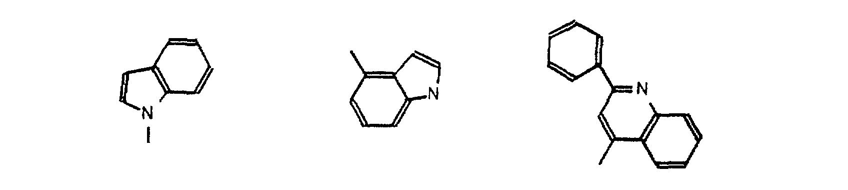 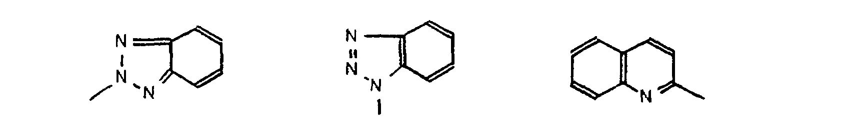 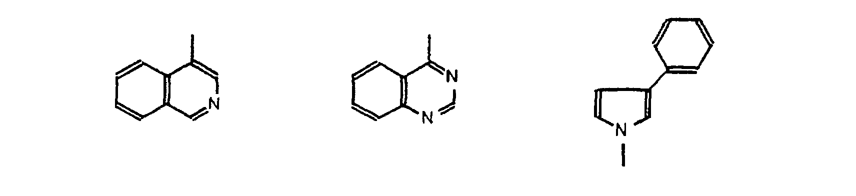 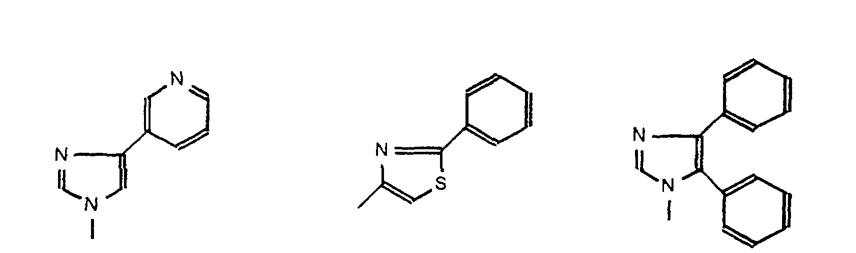 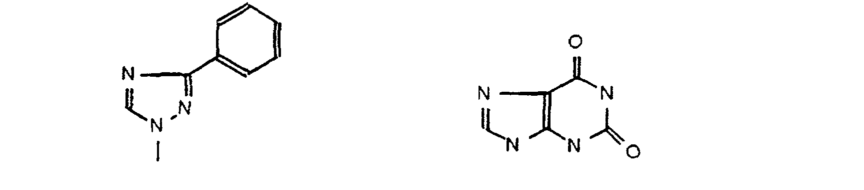 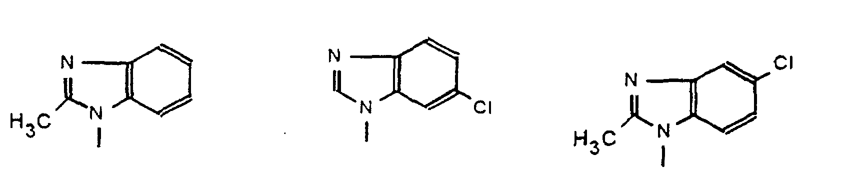 et les radicaux hétérocycliques envisagés dans les demandes de brevets européens 487411, 596802, 676409 et 680967. Ces radicaux hétérocycliques préférés peuvent être substitués par un ou plusieurs groupements fonctionnels.

Parmi les sels d'addition avec les acides, on peut citer les sels formés avec les acides acétique, propionique, trifluoroacétique, malique, tartrique, méthanesulfonique, benzènesulfonique, p-toluènesulfonique, et spécialement les acides stéariques, éthylsuccinique ou laurylsulfonique.

L'invention a tout spécialement pour objet les composés de formule (I), répondant à la formule (I), dans lesquels X représente un groupement CH₂ ou NH, R représente un radical (CH₂)ₙAr, N-(CH₂)ₙAr ou N=CH(CH₂)ₙAr, dans lequel n représente un nombre entier compris entre 1 et 6 et Ar représente un radical aryle ou hétéroaryle, éventuellement substitué, les traits pointillés représentant une double liaison éventuelle en 2(3), et Y représente un atome d'hydrogène ou le reste d'un acide organique carboxylique renfermant jusqu'à 18 atomes de carbone ainsi que leurs sels d'addition avec les acides.

L'invention a plus particulièrement pour objet les composés de formule (I), dans lesquels les traits pointillés représentent une double liaison en 2(3), ceux dans lesquels Y représente un atome d'hydrogène et ceux dans lesquels R représente un groupement (CH₂)ₙAr, n et Ar conservant la même signification que précédemment.

Parmi les composés préférés de l'invention, on peut citer tout spécialement les composés de formule (I) dans lesquels R représente un radical (CH₂)₃Ar, (CH₂)₄Ar ou (CH₂)₅Ar, et notamment les composés dans lesquels Ar représente un radical : 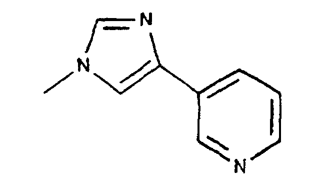 éventuellement substitué ou un radical : 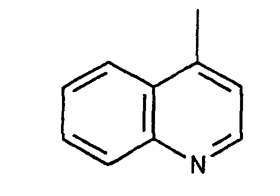 éventuellement substitué comme par exemple les composés de formule (I) dans lesquels Ar représente un radical :

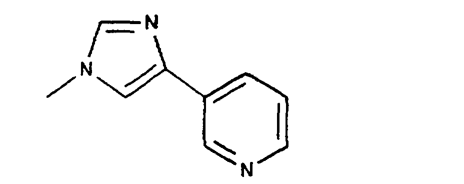

L'invention a plus particulièrement pour objet le composé de l'exemple 1.

Les produits de formule générale (I) possèdent une très bonne activité antibiotique sur les bactéries gram ^{⊕} telles que les staphylocoques, les streptocoques, les pneumocoques.

Les composés de l'invention peuvent donc être utilisés comme médicaments dans le traitement des infections à germes sensibles et notamment, dans celui des staphylococcies, telles que les septicémies à staphylocoques, staphylococcies malignes de la face ou cutanées, pyodermites, plaies septiques ou suppurantes, furoncles, anthrax, phlegmons, érysipèles et acné, staphylococcies telles que les angines aiguës primitives ou post-grippales, broncho-pneumonies, suppurations pulmonaires, les streptococcies telles que les angines aiguës, les otites, les sinusites, la scarlatine, les pneumococcies telles que les pneumonies, les bronchites ; la brucellose, la diphtérie, la gonococcie.

Les produits de la présente invention sont également actifs contre les infections dues à des germes comme Haemophilus influenzae, Rickettsies, Mycoplasma pneumoniae, Chlamydia, Legionella, Ureaplasma, Toxoplasma, ou à des germes du genre Mycobactérium.

La présente invention a donc également pour objet, à titre de médicaments et, notamment de médicaments antibiotiques, les produits de formule (I) tels que définis ci-dessus, ainsi que leurs sels d'addition avec les acides minéraux ou organiques pharmaceutiquement acceptables.

L'invention a plus particulièrement pour objet, à titre de médicaments et, notamment de médicaments antibiotiques, les produits de l'exemple 1 et les sels pharmaceutiquement acceptables.

L'invention a également pour objet les compositions pharmaceutiques renfermant comme principe actif au moins un des médicaments définis ci-dessus.

Ces compositions peuvent être administrées par voie buccale, rectale, parentérale ou par voie locale en application topique sur la peau et les muqueuses, mais la voie d'administration préférée est la voie buccale.

Elles peuvent être solides ou liquides et se présenter sous les formes pharmaceutiques couramment utilisées en médecine humaine, comme par exemple, les comprimés simples ou dragéifiés, les gélules, les granulés, les suppositoires, les préparations injectables, les pommades, les crèmes, les gels ; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Ces compositions peuvent également se présenter sous forme d'une poudre destinée à être dissoute extemporanément dans un véhicule approprié, par exemple de l'eau stérile apyrogène.

La dose administrée est variable selon l'affection traitée, le sujet en cause, la voie d'administration et le produit considéré. Elle peut être, par exemple, comprise entre 50 mg et 300 mg par jour par voie orale, chez l'adulte pour le produit de l'exemple 1.

l'invention a également pour objet un procédé caractérisé en ce que l'on soumet un composé de formule (II) : 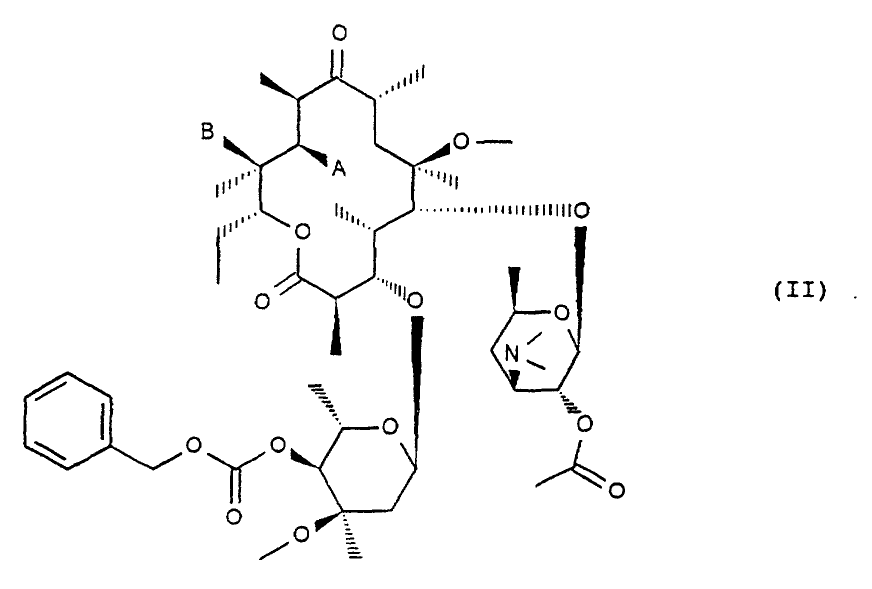 dans lequel A et B conservent leur signification précédente, à l'action d'un agent de clivage de la liaison glycoside pour libérer l'hydroxyle en 3 et obtenir un composé de formule (III) : 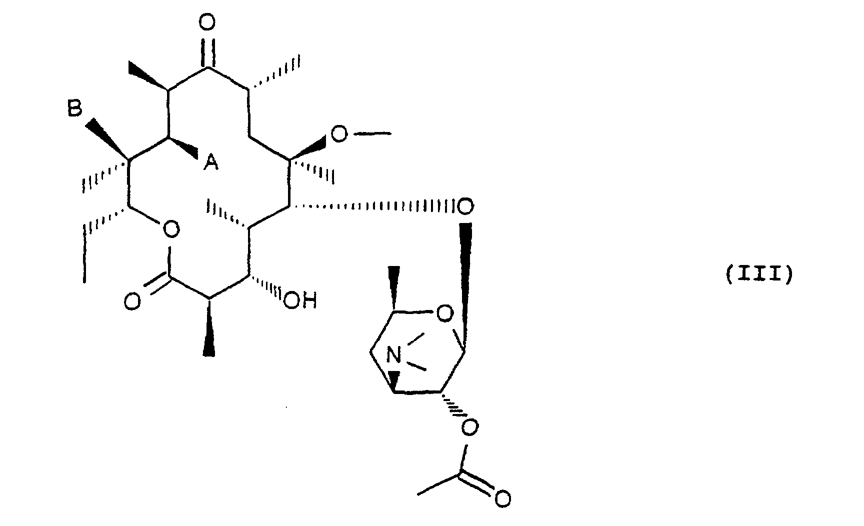 bloque l'hydroxyle en 3 sous forme de mésylate pour obtenir le composé de formule (IV) : 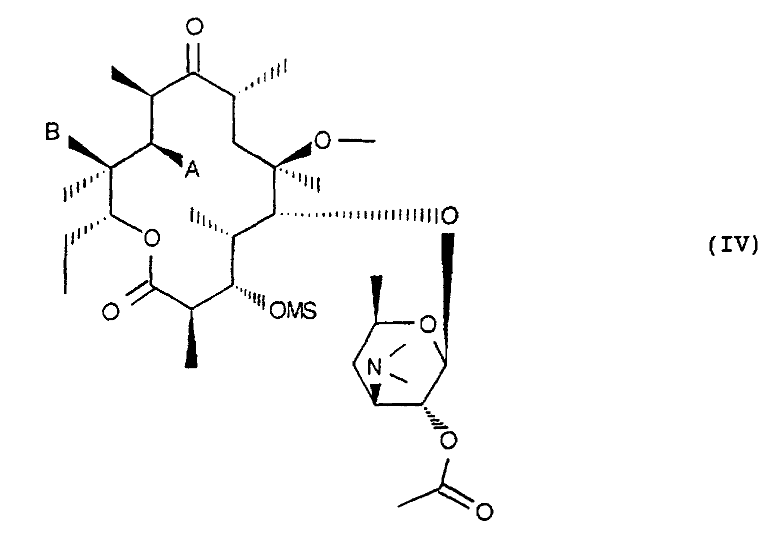 que l'on soumet à l'action d'une base pour obtenir le composé de formule (V) : 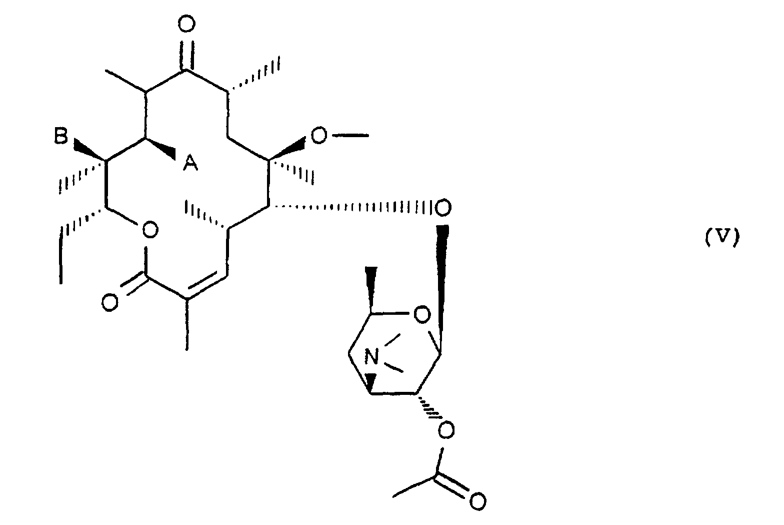 que l'on soumet, si désiré, à l'action d'un agent de clivage de l'hydroxyle en 2' pour obtenir le composé de formule (I) correspondant.

L'invention a plus particulièrement pour objet un procédé de préparation des composés de formule (I), caractérisé en ce que l'on soumet un composé de formule (II_{A}) : 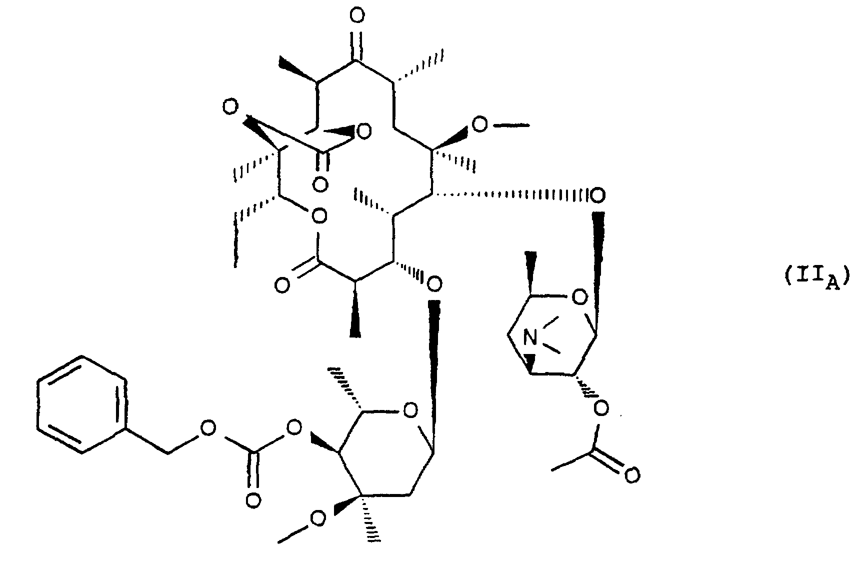 à l'action d'un agent de clivage de la liaison glycoside pour libérer l'hydroxyle en 3 et obtenir un composé de formule (III_{A}) : 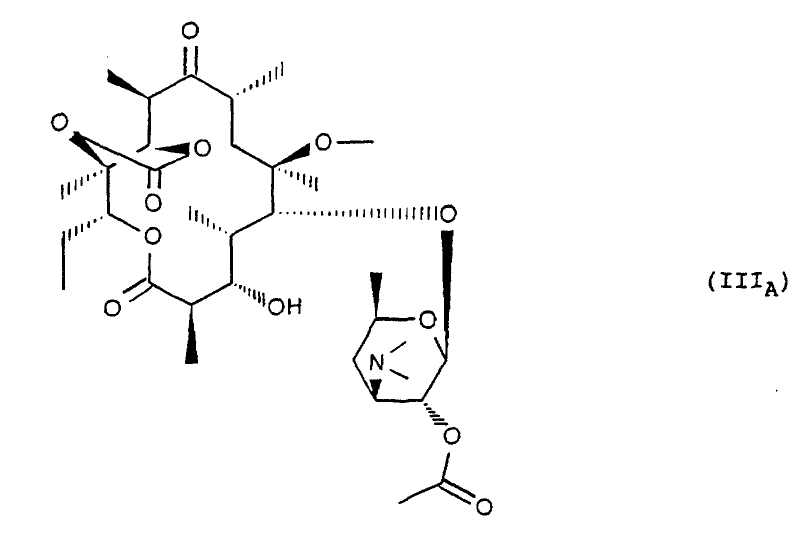 bloque l'hydroxyle en 3 sous forme de mésylate pour obtenir le composé de formule (IV_{A}) : 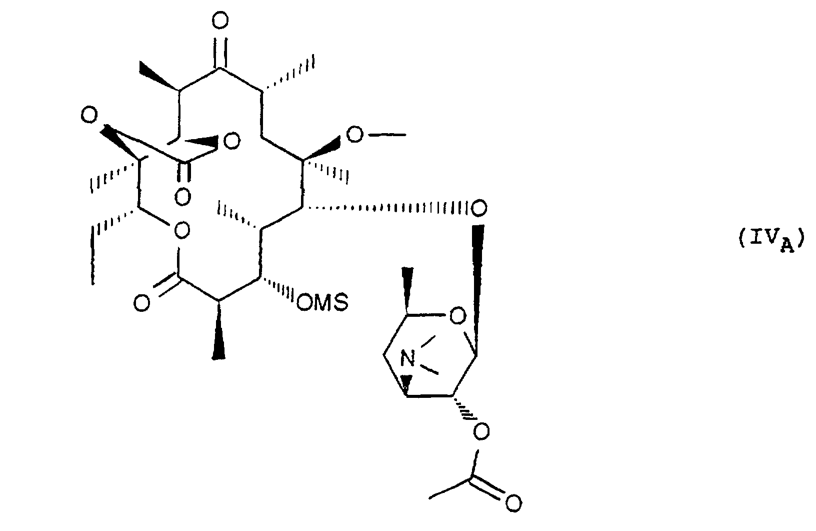 que l'on soumet à l'action d'une base pour obtenir le composé de formule (V_{A}) : 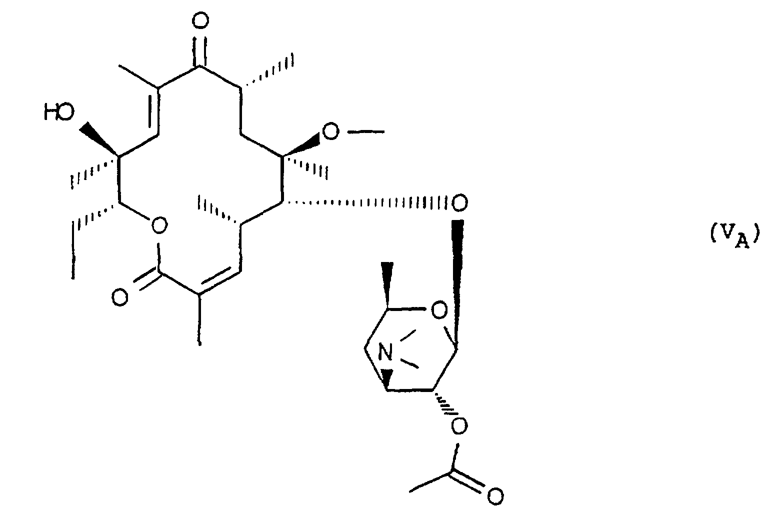 que l'on soumet à l'action du carbonydiimidazole pour obtenir le composé de formule (VI) : 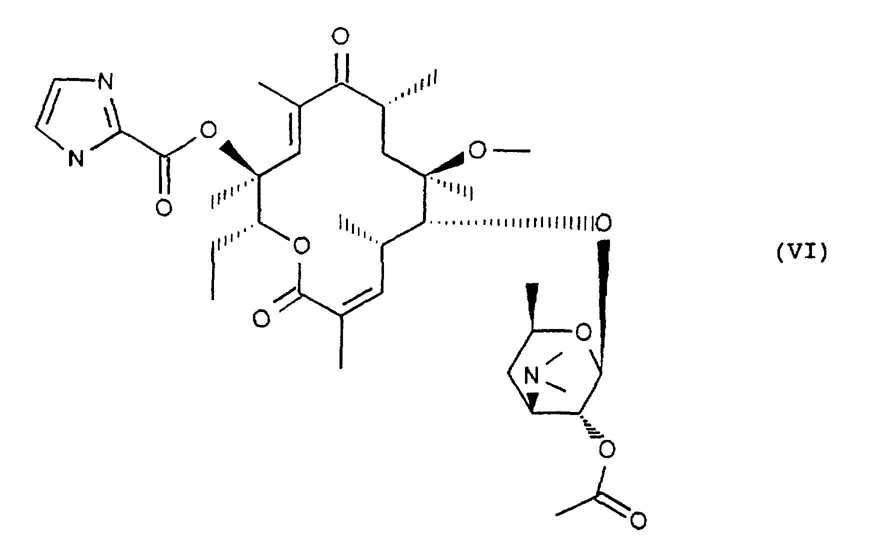 que l'on soumet à l'action du composé de formule (VII) :

RXNH₂ (VII)

dans laquelle R et X conservent leur signification précédente pour obtenir le composé de formule (I_{A}) : 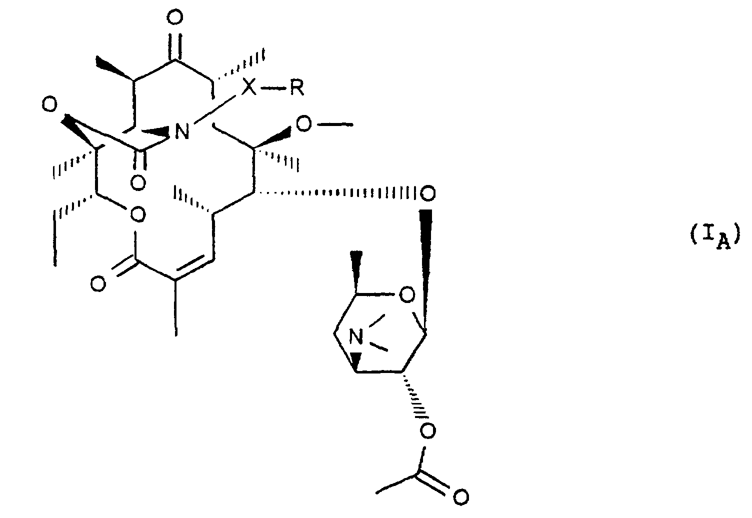 libère si désiré, l'hydroxyle en 2', pour obtenir le composé de formule (I_{B}) : 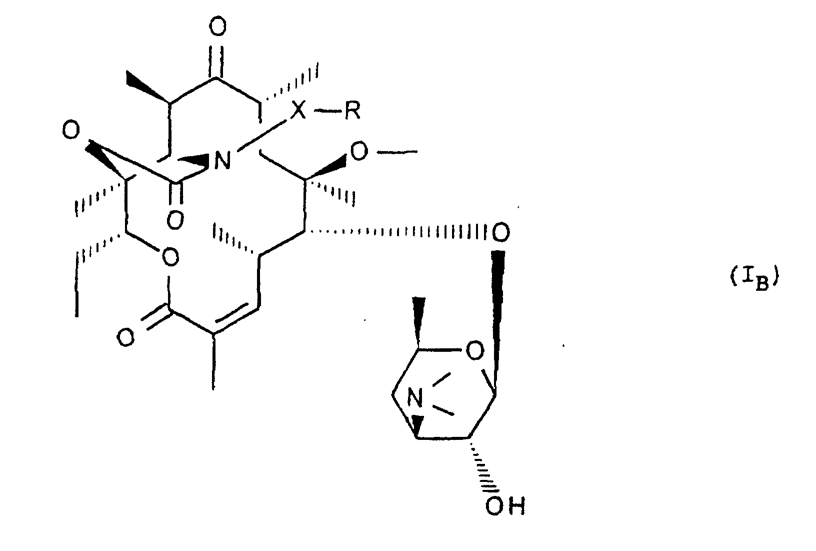 que l'on soumet, si désiré, à l'action d'un agent d'estérification de l'hydroxyle en 2', et/ou à l'action d'un agent de réduction de la double liaison en 2(3) et/ou à l'action d'un acide pour en former le sel.

Les produits de formule (I) utilisés comme produits de départ sont des produits connus qui peuvent être préparés selon le procédé décrit par BAKER et Col. dans J. Org. Chem. 1988, 53, 2340, 2345.

Dans un mode de réalisation préféré :
- l'hydrolyse du cladinose en position 3 est réalisée au moyen de l'acide chlorhydrique concentré,
- la mésylation est réalisée à l'aide de l'acide méthane-sulfonique, ou de l'un de ses dérivés par exemple l'anhydride méthanesulfonique,
- la base utilisée lors de la transformation du composé de formule (IV) en composé de formule (V) est un diazabicycloundécène, par exemple le DBU (ou 1,8-diazabicyclo[5-4-0]undec-7-ène), ou le diazabicyclononène, ou la 2,6-lutidine, ou la 2,4,6-collidine ou la tétraméthylguanidine,
- le passage du composé de formule (V) au composé de formule (VI) est réalisé à l'aide du carbonyldiimidazole,
- la réaction du composé de formule (IV) avec le composé de formule (VII) RXNH₂ a lieu en présence d'une base par exemple un diazabicycloundécène, comme le DBU, ou la diazabicyclononène ou la 2,6-butidine ou la 2,4,6-collidine ou la tétraméthylguanidine.

Les composés obtenus lors de la mise en oeuvre du procédé sont des produits nouveaux et sont en eux-mêmes un objet de la présente invention.

L'invention a donc pour objet à titre de produits chimiques nouveaux les composés de formules (III), (IV), (V) et (VI).

L'invention a plus particulièrement pour objet à titre de produits chimiques nouveaux les produits dont la préparation est donnée ci-après dans la partie expérimentale.

### EXEMPLE 1 : 2,3-didéhydro-11,12-didéoxy-3-de[(2,6-didéoxy-3-C-méthyl-3-O-méthyl-alpha-L-ribo-hexopyranosyl)oxy]6-O-méthyl-12,11-[oxycarbonyl[[4-[4-(3-pyridinyl)-1H-imidazol-1-yl]butyl]imino]]-érythromycine

### STADE A : 11,12-carbonate cyclique 2'-acétate de 3-O-de(2,6-idéoxy-3-C-méthyl-3-O-méthyl-alpha-L-ribo-hexopyranosyl)-6-O-méthyl-érythromycine

On refroidit à 5°C un mélange de 17,9 g de 11,12-carbonate cyclique 2'-acétate 4"-(phéylméthyl carbonate) de 6-O-méthyl-érythromycine et 360 ml de méthanol. On ajoute 90 ml d'une solution d'acide chlorhydrique concentrée. On laisse remonter la température à la température ambiante et laisse la solution sous agitation pendant 6 heures. On verse le milieu réactionnel sur un mélange glace ammoniaque, extrait à l'acétate d'éthyle, lave à l'eau, sèche et concentre sous pression réduite. On isole 11,62 g d'un produit que l'on cristallise dans l'éther diéthylique. on essore les cristaux obtenus, les lave et les sèche sous vide à 70°C. On obtient 7,83 g de produit attendu. F = 226∼228°C.
RMN - CDCl₃ ppm
0,87 (t) : CH₃-CO₂ ; 0,95 (d) : 4-Me ; 1,11 (d) : 8-Me ; 1,19 (d) : 10-Me ; 1,23 (d) : 2-Me ; 1,27 (d) : 5'-Me ; 1,28-1,49 : 6 et 12-Me ; ~ 1,34 et 1,73 : CH₂ en 4' ; ~ 1,49 et 1,63 : CH₂ en 7 ; ∼ 1,59 et 1,90 : CH₂ en 14 ; 1,86 (d) : 3-OH ; 1,98 (dq) : H₄ ; 2,07 (s) : OAc ; 2,26 (s) : N(Me)₂ ; 2,58 (m) : H₈ ; 2,71 (m) : H₂ et H₃' ; 2,92 (s) : 6-OMe ; 2,95 (q) : H₁₀ ; 3,48 (m) : H₃ et H₅' ; 3,70 (d) : H₅ ; 4,58 (d) : H₁' ; 4,74 (s) : H₁₁ ; 4,75 (dd) : H₂' ; 5,13 (dd) : H₁₃.

### STADE B : 11,12-carbonate cyclique 2'-acétate 3-(méthanesulfonate) de 3-O-de(2,6-idéoxy-3-C-méthyl-3-O-méthyl-alpha-L-ribo-hexopyranosyl)-6-O-méthyl-érythromycine

On refroidit à 10°C un mélange renfermant 9,20 g du produit obtenu au stade A, 60 ml de chlorure de méthylène et 7,90 g de DMAP. On ajoute 5,99 g d'anhydride méthane sulfonique. on maintient sous agitation le mélange réactionnel à la température ambiante pendant 17 h 30. On verse le mélange réactionnel sur de la glace, extrait au chlorure de méthylène, lave à l'eau puis à l'eau salée, sèche sur sulfate de sodium anhydre et concentre sous pression réduite. On isole 11,078 g du produit attendu. F = 194∼196°C.
RMN CDCl₃ ppm
0,89 (t) : CH³-CH₂ ; 1,00 (d) : 4-Me ; 1,12 (d) : 8-Me ; 1,19 (d) : 10-Me ; 1,21 (d) : 5-Me ; 1,27 (d) : 2-Me ; 1,48-1,34 : 6 et 12-Me ; ~1,48 et 1,57 : CH₂ en 7 ; ∼1,25 et 1,70 : CH₂ en 4' ; ∼1,62 et ∼1,91 : CH₂ en 14 ; 2,02 (dq) : H₄ ; 2,06 (s) : OAc ; 2,25 (s) : N(Me)₂ ; 2,63 (m) : H₈ ; 2,72 (m) : H₃' ; 2,93 (ql) : H₁₀ ; 2,99 : 6-OMe ; 2,99 (masqué) : H₂ ; 3,13 (s) : OSO₂Me ; 3,57 (m) : H₅' ; 4,01 (d,J = 5) : H₅ ; 4,50 (d) : H₁' ; 4,68 (sl) : H₁₁ ; ~4,71 (dd) : H₂' ; 4,74 (d) : H₃ ; 5,10 (dd) : H₁₃.

### STADE C : 2'-acétate de 11-déoxy-3-de[(2,6-didéoxy-3-C-méthyl-3-O-méthyl-alpha-L-ribo-hexopyranosyl)oxy]-6-O-méthyl-2,3,10,11-tétradehydro-érythromycine

On porte au reflux pendant 6 h 30, un mélange de 9,56 g du produit obtenu au stade B, 95 ml d'acétone et 15,5 ml de DBU. On verse le milieu réactionnel sur un mélange de glace et d'eau. On filtre le précipité, le dissout dans l'acétate d'éthyle, sèche sur sulfate de sodium, et concentre sous pression réduite. On isole 5,45 g de produit attendu brut que l'on empâte dans l'éther diéthylique. On filtre et sèche à 70°C sous vide. On isole 2,781 g de produit attendu. F = 156∼158°C.
RMN CDCl₃ ppm
1,02 (t) : CH₃-CH₂ ; 1,14 (d) : 4-Me ; 1,23 (d) : 8-Me ; 1,27 (d) : 5'-Me ; 1,30-1,34 : 6 et 12-Me ; 1,78-2,02-2,04 : 2-Me-10-Me et 2'-OAc ; ∼1,35 et 1,75 : CH₂ en 4' ; ~1,49 et 1,99 : CH₂ en 7 ; ~1,62 et 1,83 : CH₂ en 14 ; ∼2,70 : H₃' et H₄ ; 2,27 (s) : N(Me)₂ ; 3,01 (m) : H₈ ; 3,07 (s) 6-OMe ; 3,52 (m) : H₅' ; 3,70 (d) : H₃ ; 4,44 (d) : H₁' ; 4,76 (dd) : H₂' ; 4,81 (dd) : H₁₃ ; 6,28 (s,l) : H₁₁ ; 7,01 (dl) : H₃.

### STADE D : 2,3-didéhydro-11,12-didéoxy-3-de[(2,6-didéoxy-3-C-méthyl-3-O-méthyl-alpha-L-ribo-hexopyranosyl)oxy]-6-O-méthyl-12,11-[oxycarbonyl[[4-[4-(3-pyridinyl)-1H-imidazol-1-yl]-butyl]imino]]-érythromycine

On maintient sous agitation 507 mg de produit obtenu au stade C, 6 ml de THF, 22,4 µl de DBU et 243 mg de CDI. On obtient une solution que l'on ajoute dans un mélange de 345 mg d'aminobutylimidazolpyridine et de 4 ml de chlorure de méthylène. On ajoute une goutte de DBU et maintient le mélange réactionnel sous agitation pendant 8,5 jours à 0°C. On ajoute de l'acétate d'éthyle au milieu réactionnel en laissant remonter la température à la température ambiante, lave à l'eau, à l'eau ammoniaquée, puis à l'eau. On extrait les phases aqueuses à l'acétate d'éthyle, rassemble les phases organiques, sèche sur sulfate de sodium anhydre et concentre sous pression réduite. On isole 694 mg d'un produit que l'on dissout dans 7 ml de méthanol. On porte la solution au reflux, et concentre sous pression réduite. On isole 639 mg de produit attendu brut que l'on chromatographie sur silice en éluant avec le mélange acétate d'éthyle triéthylamine 90-10. On isole 292 mg de cristaux, que l'on empâte dans l'éther diéthylique. On essore, lave et sèche à 50°C sous pression réduite le produit obtenu. On obtient 170 mg de produit que l'on dissout dans de l'acétate d'éthyle, lave à l'eau ammoniaquée, sèche sur sulfate de sodium anhydre et concentre. On isole 140 mg de cristaux qui sont empâtés dans de l'acétate d'éthyle, lavés et séchés sous pression réduite à 60°C. On obtient 94 mg de produit attendu. F = 196~198°C. RMN CDCl₃ ppm
0,98 (t) : CH₃-CH₂ ; 1,01 (d) : 10-Me ; 1,15 (d) : 8-Me ; 1,25 (d) : 5'-Me ; 1,28 (d) : 4-Me ; 1,30 et 1,48 : 6 et 12-Me ; 1,89 (sl) : 2-Me ; 2,27 (s) : N-(CH₃)₂ ; 2,47 (m) : H₃' ; 2,64 (m) : H₈ ; 2,76 (m) : H₄ ; 2,76 (s) : 6-OMe ; 3,00 (q) : H₁₀ ; 3,16 (dd) : H₂' ; 3,52 : H₅ ; 3,60 : H₅ ; 3,27 H₁₁ ; 3,52 (m), 4,04 (m) : les CH₂-N-C= ; 4,41 (d) : H₁' ; 4,71 (dd) : H₁₃ ; 6,69 (dl) : H₃ ; 7,27 (dm) : H5 - 8,10 (dt) : H₄ - 8,45 (dd) : H₆ - 9,01 (dd) : H₂ (pyridine) ; 7,40 (d) - 7,56 (d) (les H imidazoles).
Variante du procédé de préparation de l'invention, le produit (IV) à savoir le 2'-acétate de 11-déoxy-3-de[(2,6-didéoxy-3-C-méthyl-3-O-méthyl-alpha-L-ribo-hexopyranosyl)oxy]-6-O-méthyl-2,3,10,11-tétradéhydro-érythromycine peut être également préparé comme suit :

### STADE A : 3-O-de(2,6-didéoxy-3-C-méthyl-3-O-méthyl-alpha-L-ribo-hexopyranosyl)11-déoxy-6-O-méthyl-érythromycine

On porte au reflux un mélange de 5 g de 3-O-de(2,6-didéoxy-3-C-méthyl-3-O-méthyl-alpha-L-ribo-hexopyranosyl)-6-O-méthyl-érythromycine, 85 cm³ d'acétate d'éthyle, 2,8 g de carbonate d'éthylène et 1,1 g de carbonate de potassium, sèche à 160°C. On maintient l'agitation pendant 80 heures. On filtre et évapore sous pression réduite, on obtient 8,54 g du produit que l'on chromatographie sur silice en éluant avec le mélange acétate d'éthyle/triéthylamine 96/4. On obtient 3,07 g de produit recherché.

### STADE B : 2'-acétate de 3-O-de(2,6-didéoxy-3-C-méthyl-3-O-méthyl-alpha-L-ribo-hexopyranosyl)11-déoxy-6-O-méthylérythromycine

On maintient sous agitation pendant une nuit à la température ambiante 1,0612 g de produit du stade A, 15 cm³ d'acétate d'éthyle et 0,2 cm³ d'anhydride acétique. On ajoute 20 cm³ d'eau et 4 cm³ d'ammoniaque. On extrait à l'acétate d'éthyle, lave à l'eau, sèche et concentre. On obtient 0,9426 g de produit recherché.

### STADE C : 2'-acétate 3-(méthanesulfonate) de 11-déoxy-3-O-de(2,6-didéoxy-3-C-méthyl-3-O-méthyl-alpha-L-ribohexopyranosyl)-6-O-méthyl-érythromycine

On ajoute à 10°C, 194 µl de chlorure de mésyle dans une solution renfermant de 600 mg de produit du stade B, 3 cm³ de chlorure de méthylène et 404 µl de pyridine. On laisse remonter à la température ambiante et maintient l'agitation pendant 8 heures. On verse sur de la glace, extrait au chlorure de méthylène, lave à l'eau, sèche, filtre et concentre. On obtient après chromatographie et cristallisation dans l'éther, 0,675 g de produit recherché.

### STADE D : 2'-acétate de 11-déoxy-3-de[(2,6-didéoxy-3-C-méthyl-3-O-méthyl-alpha-L-ribo-hexopyranosyl) oxy] -6-O-méthyl-2,3,10,11-tétradéhydro-érythromycine

On porte au reflux pendant 24 heures un mélange de 100 mg de produit préparé au stade précédent, 255 µl de DBU et 6 ml d'acétone. On verse dans l'eau, extrait à l'acétate d'éthyle, lave à l'eau, sèche, filtre et concentre sous pression réduite et chromatographie sur silice, en éluant avec le mélange acétate d'éthyle-triéthylamine 98-2. On obtient ainsi 51 mg de produit recherché.

### EXEMPLE DE COMPOSITION PHARMACEUTIQUE

On a préparé des composés renfermant :

| | |
|---|---|
| Produit de l'exemple 1 | 150 mg |
| Excipient q.s.p. | 1 g |

Détail de l'excipient : amidon, talc, stéarate de magnésium

### ETUDE PHARMACOLOGIOUE DES PRODUITS DE L'INVENTION

### Méthode des dilutions en milieu liquide

On a préparé une série de tubes dans lesquels on répartit une même quantité de milieu nutritif stérile. On distribue dans chaque tube des quantités croissantes du produit à étudier, puis chaque tube est ensemencé avec une souche bactérienne. Après incubation de vingt-quatre heures à l'étuve à 37°C, l'inhibition de la croissance est appréciée par transillumination de ce qui permet de déterminer les concentrations minimales inhibitrices (C.M.I.) exprimées en microgrammes/cm³.

Les résultats suivants ont été obtenus :

| Souches bactériennes à GRAM+ | |
|---|---|
| Produits | Ex. 1 |
| Staphylococcus aureus 011UC4 | 0,08 |
| Staphylococcus epidermidis 012GO11I | 0,15 |
| Streptococcus pyogenes groupe A 02A1UC1 | ≤ 0,02 |
| Streptococcus agalactiae groupe B 02B1HT1 | ≤ 0,02 |
| Streptococcus faecalis groupe D 02D2UC1 | ≤ 0,02 |
| Streptococcus faecium groupe D 02D3HT1 | ≤ 0,02 |
| Streptococcus sp groupe G 02G0GR5 | ≤ 0,02 |
| Streptococcus mitis 02mitCB1 | ≤ 0,02 |
| Streptococcus mitis 02mitGR16I | ≤ 0,02 |
| Streptococcus agalactiae groupe B 02B1SJ1 | 0,08 |
| Streptococcus pneumoniae 030ROIi | ≤ 0,02 |

De plus, le produit de l'exemple 1 a montré une activité intéressante sur les souches bactériennes à gram^{⊖} suivantes : Haemophilus Influenzae 351HT3, 351CB12, 351CA1 et 351GR6.

## Revendications

1. Les composés de formule (I) : 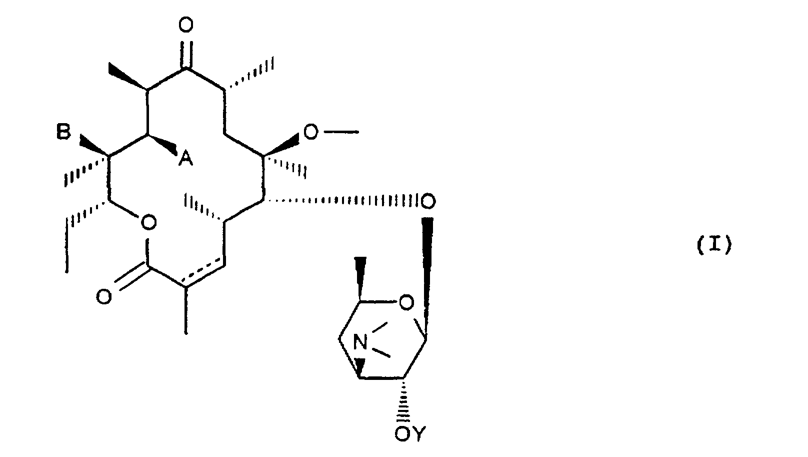 dans lesquels :
- ou bien A et B représentent un radical OH,
- ou bien B représente un radical OH et A forme avec le carbone qui le porte et le carbone en 10 une double liaison,
- ou bien A et B forment ensemble un radical carbonate,
- ou bien A et B forment ensemble avec les atomes de carbone qui les portent un cycle : 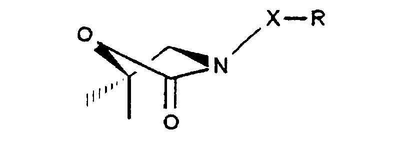
dans lesquels X représente un groupement CH₂, NH ou SO₂, R représente un radical (CH₂)ₙAr, N-(CH₂)ₙAr ou N=CH(CH₂)ₙAr, dans lequel n représente un nombre entier compris entre 1 et 6 et Ar représente un radical aryle ou hétéroaryle, éventuellement substitué, les traits pointillés représentant une double liaison éventuelle en 2(3), et Y représente un atome d'hydrogène ou le reste d'un acide organique carboxylique renfermant jusqu'à 18 atomes de carbone ainsi que leurs sels d'addition avec les acides.

2. Les composés de formule (I) tels que définis à la revendication 1, répondant à la formule (I), dans lesquels X représente un groupement CH₂ ou NH, R représente un radical (CH₂)ₙAr, N-(CH₂)ₙAr ou N=CH(CH₂)ₙAr, dans lequel n représente un nombre entier compris entre 1 et 6 et Ar représente un radical aryle ou hétéroaryle, éventuellement substitué, les traits pointillés représentant une double liaison éventuelle en 2(3), et Y représente un atome d'hydrogène ou le reste d'un acide organique carboxylique renfermant jusqu'à 18 atomes de carbone ainsi que leurs sels d'addition avec les acides.

3. Les composés de formule (I) définis à la revendication 1 ou 2, dans lesquels les traits pointillés représentent une double liaison en 2(3).

4. Les composés de formule (I) tels que définis à la revendication 1, 2 ou 3, dans lesquels Y représente un atome d'hydrogène.

5. Les composés de formule (I) tels que définis à l'une quelconque des revendications 2 à 4, dans lesquels R représente un groupement (CH₂)ₙAr, n et Ar conservant la même signification que dans la revendication 1.

6. Les composés de formule (I) définis à l'une quelconque des revendications 2 à 5, dans lesquels R représente un radical (CH₂)₃Ar, (CH₂)₄Ar ou (CH₂)₅Ar.

7. Les composés de formule (I) tels que définis à l'une quelconque des revendications 2 à 6, dans lesquels Ar représente un radical : 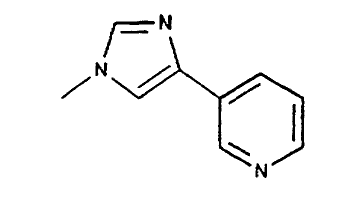 éventuellement substitué ou un radical : 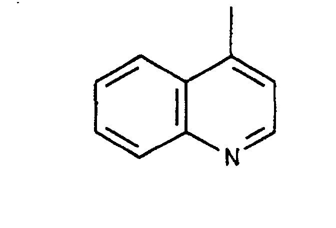 éventuellement substitué

8. Les composés de formule (I) tels que définis à la revendication 7, dans lesquels Ar représente un radical :

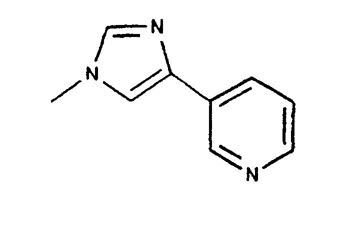

9. Les composés de formule (I) définis à la revendication 1, dont le nom suit :
2,3-didéhydro-11,12-didéoxy-3-de[(2,6-didéoxy-3-C-méthyl-3-O-méthyl-alpha-L-ribo-hexopyranosyl) oxy]-6-O-méthyl-12,11-[oxycarbonyl[[4-[4-(3-pyridinyl)-1H-imidazol-1-yl]butyl]-imino]]-érythromycine.

10. A titre de médicaments les composés de formule (I) définis à l'une quelconque des revendications 1 à 8, ainsi que leurs sels d'addition avec les acides pharmaceutiquement acceptables.

11. A titre de médicament, le composé défini à la revendication 9, ainsi que ses sels d'addition avec les acides pharmaceutiquement acceptables.

12. Les compositions pharmaceutiques renfermant comme principe actif au moins un médicament défini à la revendication 10 ou 11.

13. Procédé de préparation des composés de formule (I) définis à la revendication 1, **caractérisé en ce que** l'on soumet un composé de formule (II) : 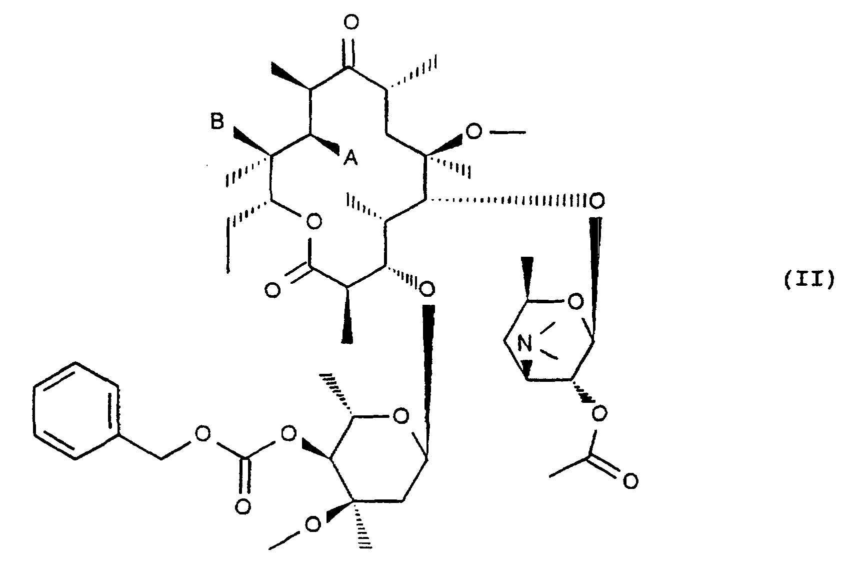 dans lequel A et B conservent leur signification précédente, à l'action d'un agent de clivage de la liaison glycoside pour libérer l'hydroxyle en 3 et obtenir un composé de formule (III) : 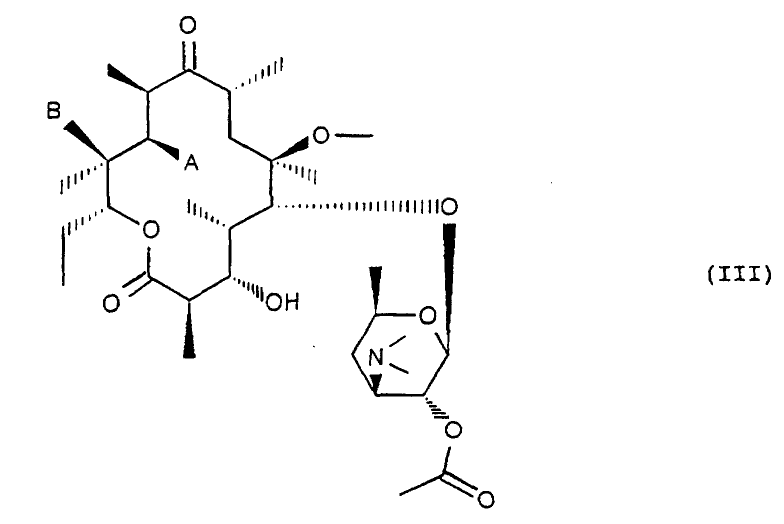 bloque l'hydroxyle en 3 sous forme de mésylate pour obtenir le composé de formule (IV) : 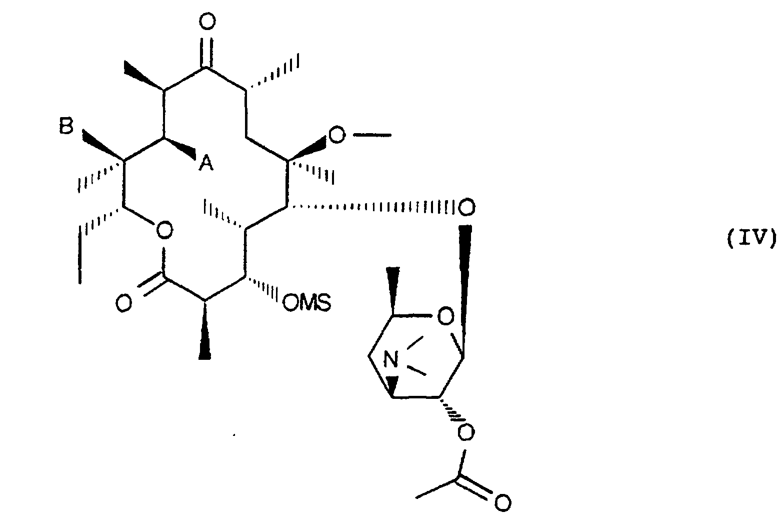 que l'on soumet à l'action d'une base pour obtenir le composé de formule (V) : 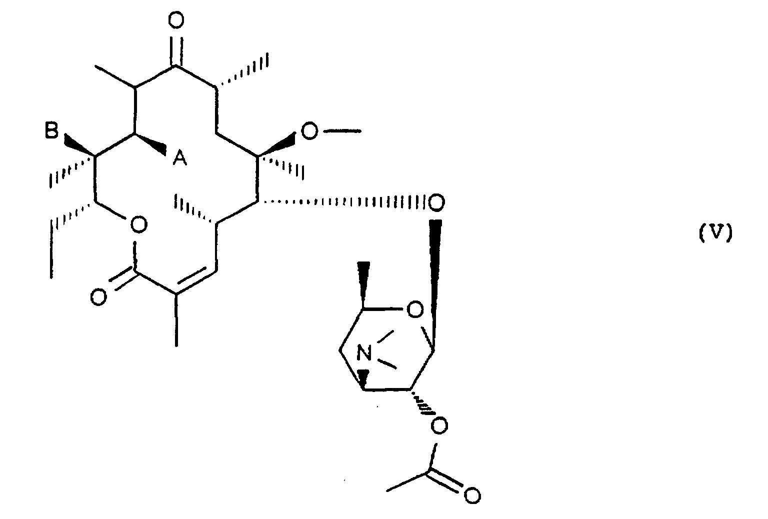 que l'on soumet, si désiré, à l'action d'un agent de clivage de l'hydroxyle en 2' pour obtenir le composé de formule (I) correspondant.

14. Procédé de préparation des composés de formule (I) définis à la revendication 1, **caractérisé en ce que** l'on soumet un composé de formule (II_{A}) : 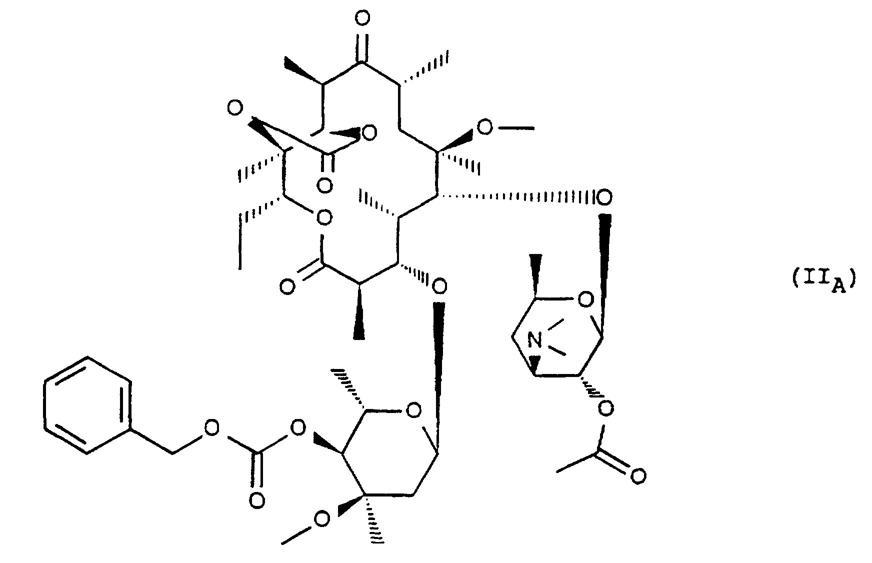 à l'action d'un agent de clivage de la liaison glycoside pour libérer l'hydroxyle en 3 et obtenir un composé de formule (III_{A}) : 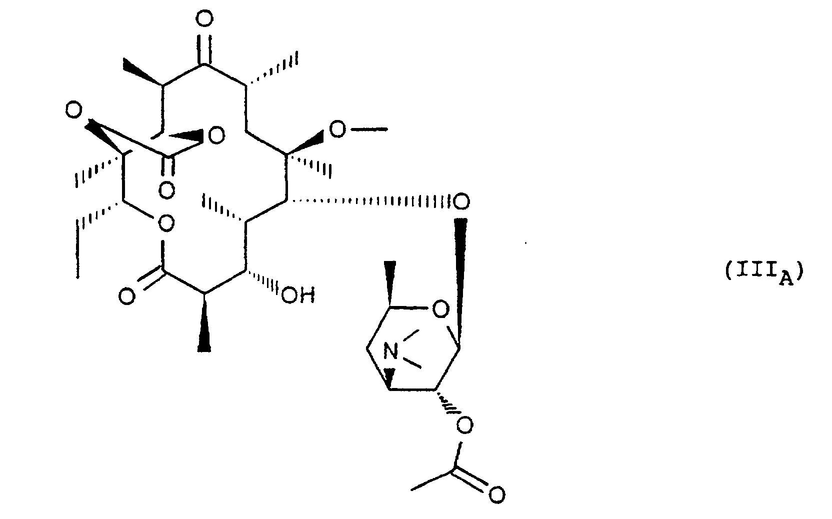 bloque l'hydroxyle en 3 sous forme de mésylate pour obtenir le composé de formule (IV_{A}) : 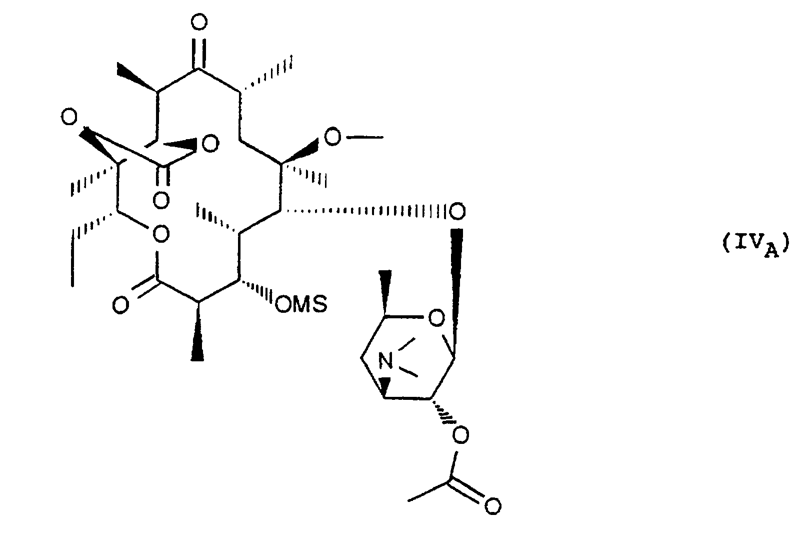 que l'on soumet à l'action d'une base pour obtenir le composé de formule (V_{A}) : 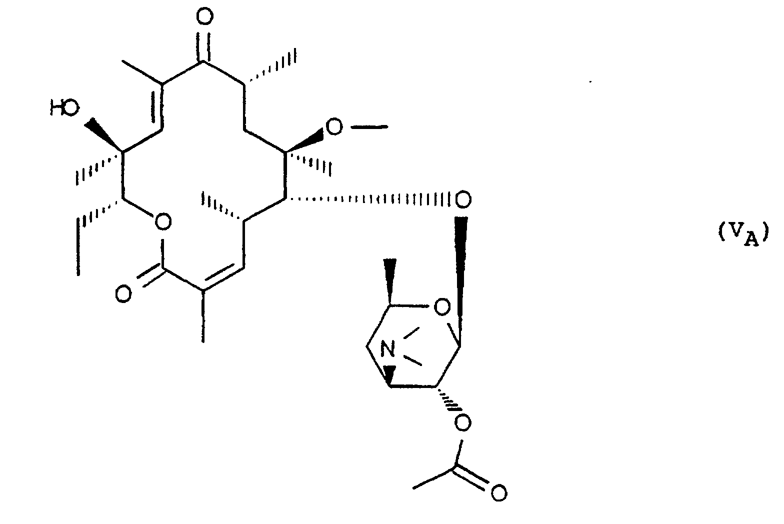 que l'on soumet à l'action du carbonydiimidazole pour obtenir le composé de formule (VI) : 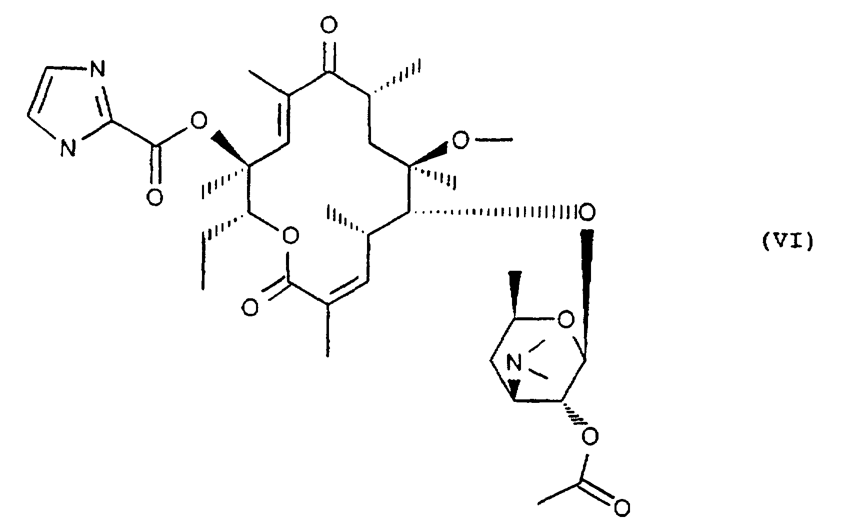 que l'on soumet à l'action du composé de formule (VII) :
RXNH₂ (VII)
dans laquelle R et X conservent leur signification précédente pour obtenir le composé de formule (I_{A}) : 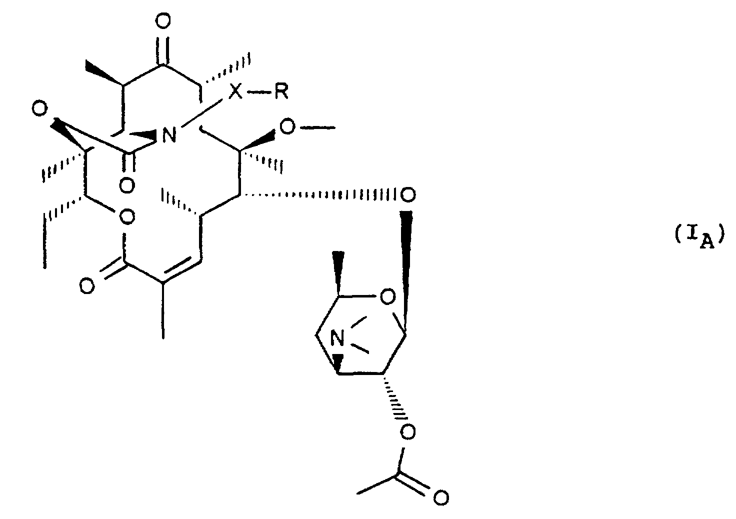 libère si désiré, l'hydroxyle en 2', pour obtenir le composé de formule (I_{B}) : 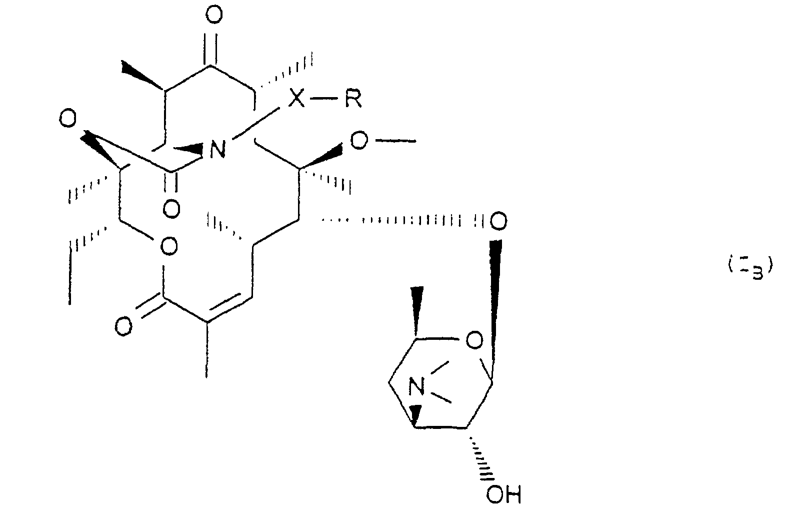 que l'on soumet, si désiré, à l'action d'un agent d'estérification de l'hydroxyle en 2', et/ou à l'action d'un agent de réduction de la double liaison en 2(3) et/ou à l'action d'un acide pour en former le sel.

15. Les composés de formules (IV), (V) et (VI) définis à la revendication 13 ou 14.

16. Les produits chimiques définis à la revendication 15 suivants :
- 11,12-carbonate cyclique 2'-acétate 3-(méthanesulfonate) de 3-O-de(2,6-didéoxy-3-C-méthyl-3-O-méthyl-alpha-L-ribohexopyranosyl)-6-O-méthyl-érythromycine,
- 2'-acétate de 11-déoxy-3-de[(2,6-didéoxy-3-C-méthyl-3-O-méthyl-alpha-L-ribo-hexopyranosyl)oxy]-6-O-méthyl-2,3,10,11-tétradéhydro-érythromycine.

## Claims

1. The compounds of formula (I): 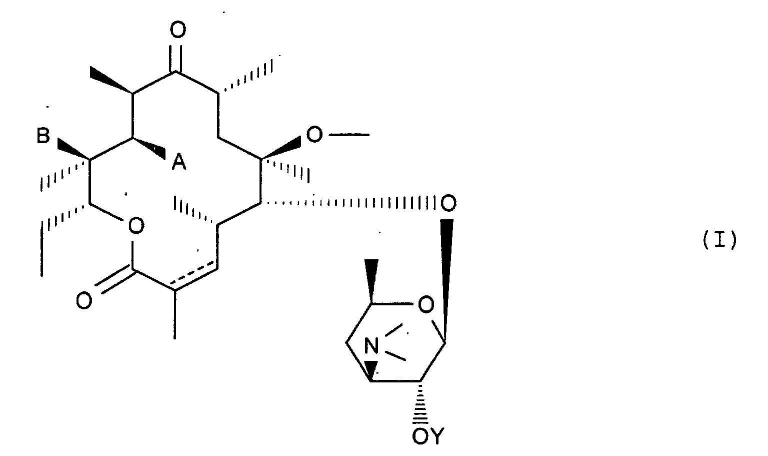 in which:
- either A and B represent an OH radical,
- or B represents an OH radical and A together with the carbon which carries it and the carbon in position 10 forms a double bond,
- or A and B together form a carbonate radical,
- or A and B together with the carbon atoms which carry it form a ring: 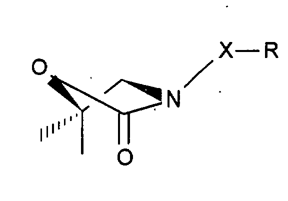 in which X represents a CH₂, NH or SO₂ group, R represents a (CH₂)ₙAr, N-(CH₂)ₙAr or N=CH(CH₂)ₙAr radical, in which n represents an integer comprised between 1 and 6 and Ar represents an aryl or heteroaryl radical, optionally substituted, the dotted lines representing an optional double bond in position 2(3), and Y represents a hydrogen atom or the remainder of an organic carboxylic acid containing up to 18 carbon atoms as well as their addition salts with acids.

2. The compounds of formula (I) as defined in claim 1, corresponding to formula (I), in which X represents a CH₂ or NH group, R represents a (CH₂)ₙAr, N-(CH₂)ₙAr or N=CH(CH₂)ₙAr radical, in which n represents an integer comprised between 1 and 6 and Ar represents an aryl or heteroaryl radical, optionally substituted, the dotted lines representing an optional double bond in position 2(3), and Y represents a hydrogen atom or the remainder of an organic carboxylic acid containing up to 18 carbon atoms as well as their addition salts with acids.

3. The compounds of formula (I) defined in claim 1 or 2, in which the dotted lines represent a double bond in position 2(3).

4. The compounds of formula (I) as defined in claim 1, 2 or 3, in which Y represents a hydrogen atom.

5. The compounds of formula (I) as defined in any one of claims 2 to 4, in which R represents a (CH₂)ₙAr group, n and Ar retaining the same meaning as in claim 1.

6. The compounds of formula (I) defined in any one of claims 2 to 5, in which R represents a (CH₂)₃Ar, (CH₂)₄Ar or (CH₂)₅Ar radical.

7. The compounds of formula (I) as defined in any one of claims 2 to 6, in which Ar represents a 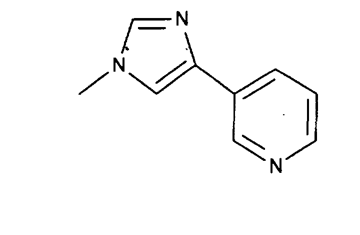 radical, optionally substituted or a 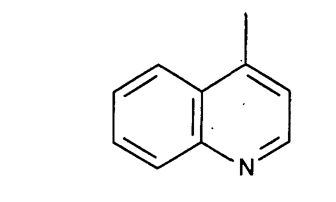 radical, optionally substituted

8. The compounds of formula (I) as defined in claim 7, in which Ar represents a 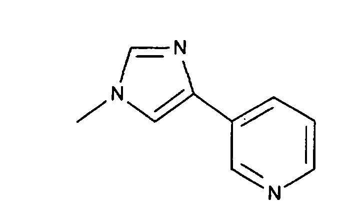 radical.

9. The compounds of formula (I) defined in claim 1, the names of which follow:
2,3-didehydro-11,12-dideoxy-3-de[(2,6-dideoxy-3-C-methyl-3-O-methyl-alpha-L-ribo-hexopyranosyl) oxy]-6-O-methyl-12,11-[oxycarbonyl[[4-[4-(3-pyridinyl)-1H-imidazol-1-yl]butyl]imino]]-erythromycin.

10. As medicaments the compounds of formula (I) defined in any one of claims 1 to 8, as well as their addition salts with pharmaceutically acceptable acids.

11. As a medicament, the compound defined in claim 9, as well as its addition salts with pharmaceutically acceptable acids.

12. The pharmaceutical compositions containing at least one medicament defined in claim 10 or 11 as active ingredient.

13. Process for the preparation of the compounds of formula (I) defined in claim 1, **characterized in that** a compound of formula (II): 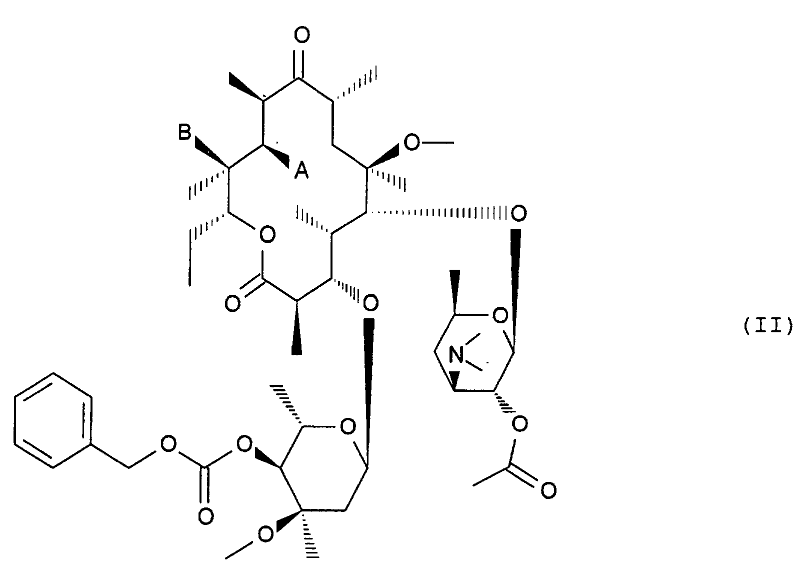 in which A and B retain their previous meaning, is subjected to the action of a cleavage agent of the glycoside bond in order to release the hydroxyl in position 3 and to obtain a compound of formula (III): 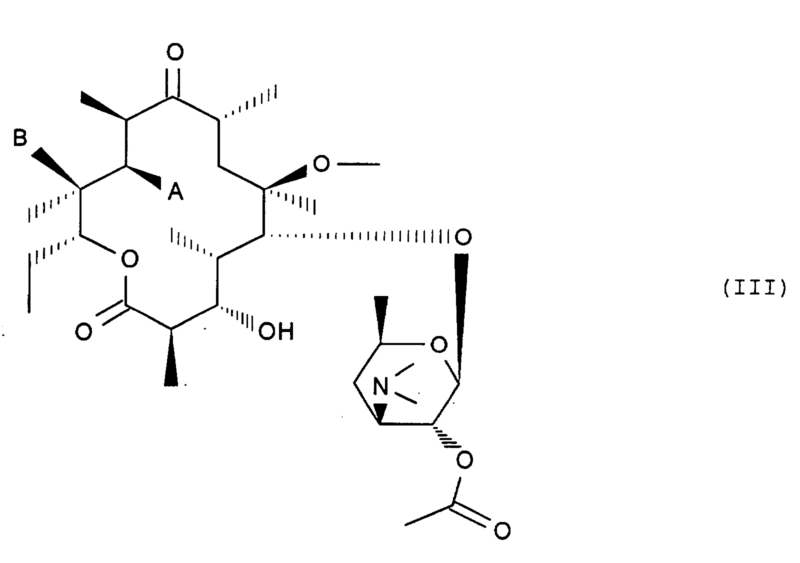 the hydroxyl in position 3 is blocked in the form of the mesylate in order to obtain the compound of formula (IV): 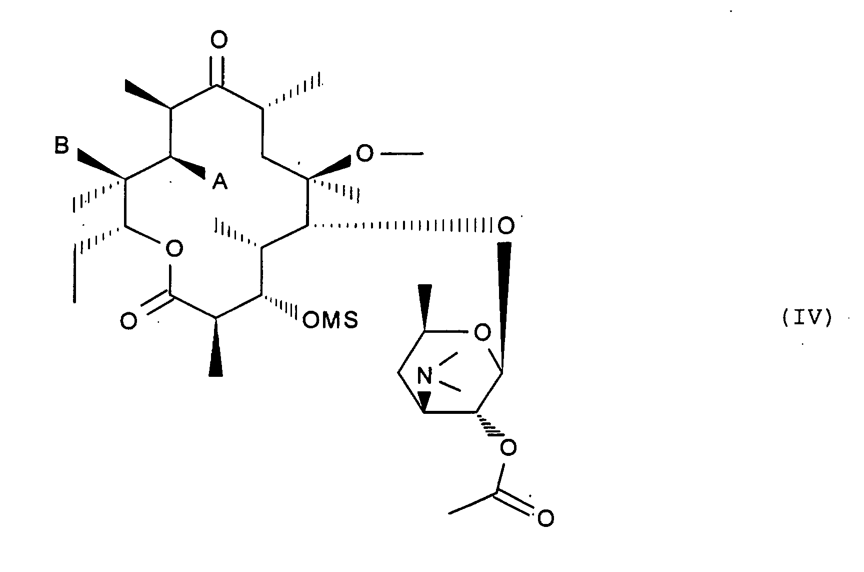 which is subjected to the action of a base in order to obtain the compound of formula (V): 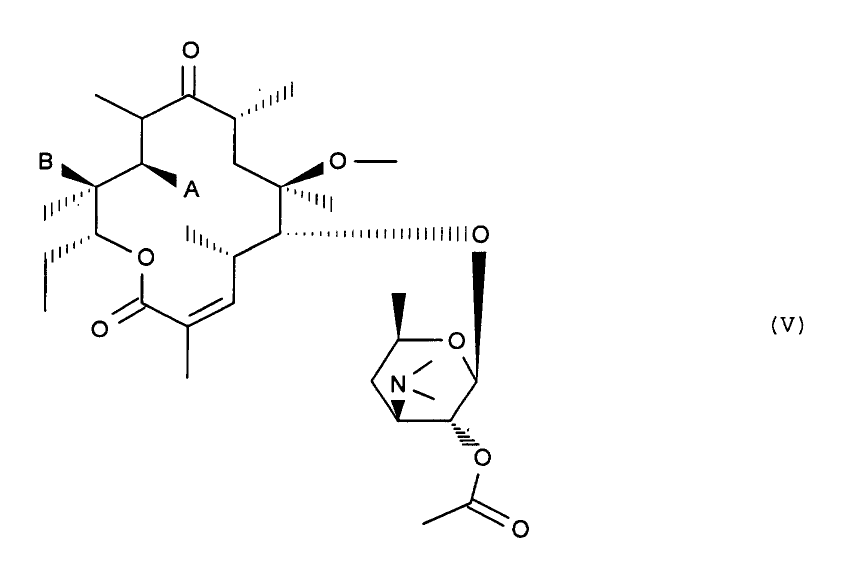 which is subjected, if desired, to the action of a cleavage agent of the hydroxyl in position 2' in order to obtain the corresponding compound of formula (I).

14. Process for the preparation of the compounds of formula (I) defined in claim 1, **characterized in that** a compound of formula (II_{A}) : 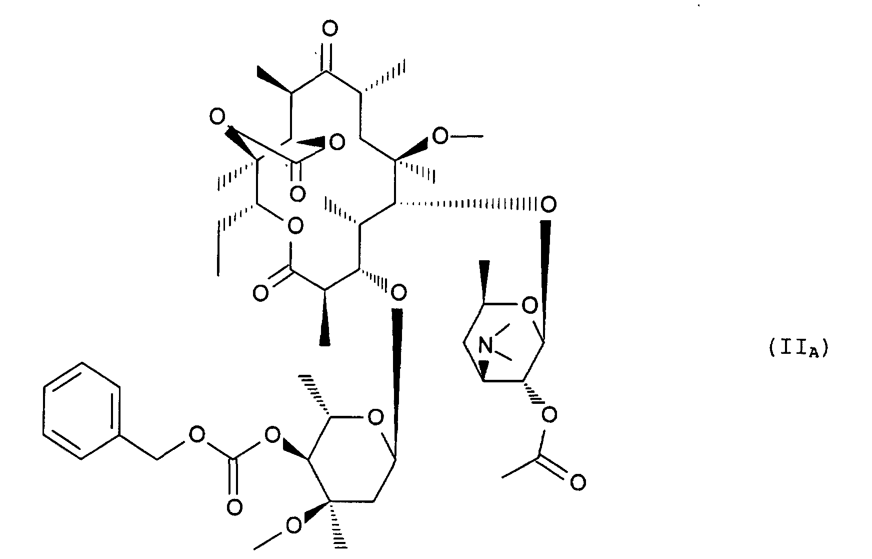 is subjected to the action of a cleavage agent of the glycoside bond in order to release the hydroxyl in position 3 and to obtain a compound of formula (III_{A}): 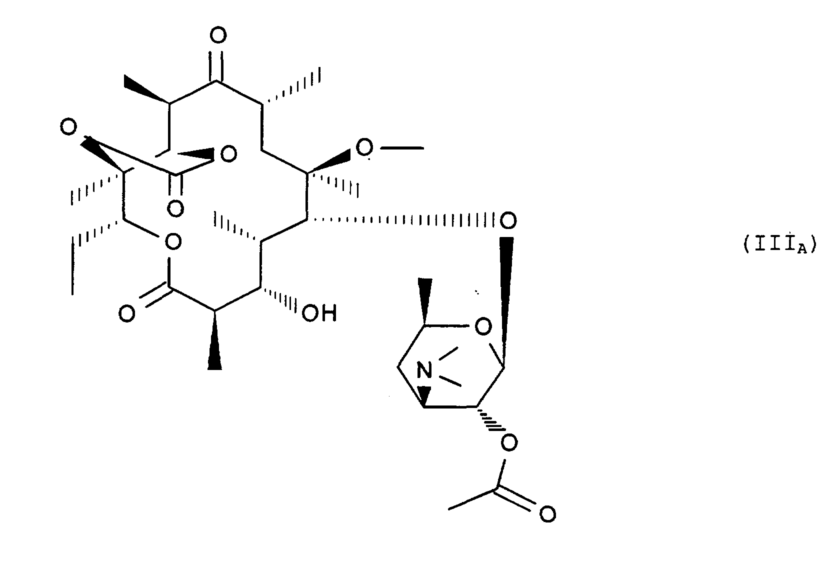 the hydroxyl in position 3 is blocked in the form of the mesylate in order to obtain the compound of formula (IV_{A}) : 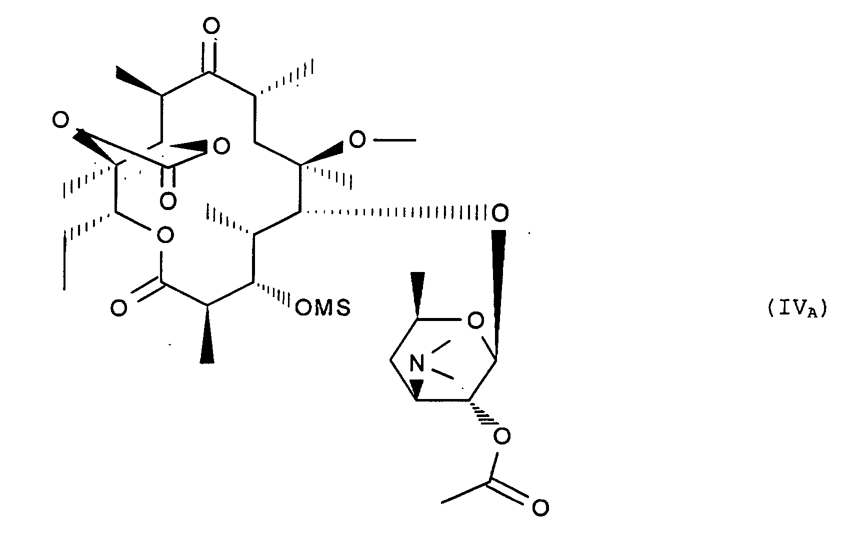 which is subjected to the action of a base in order to obtain the compound of formula (V_{A}) : 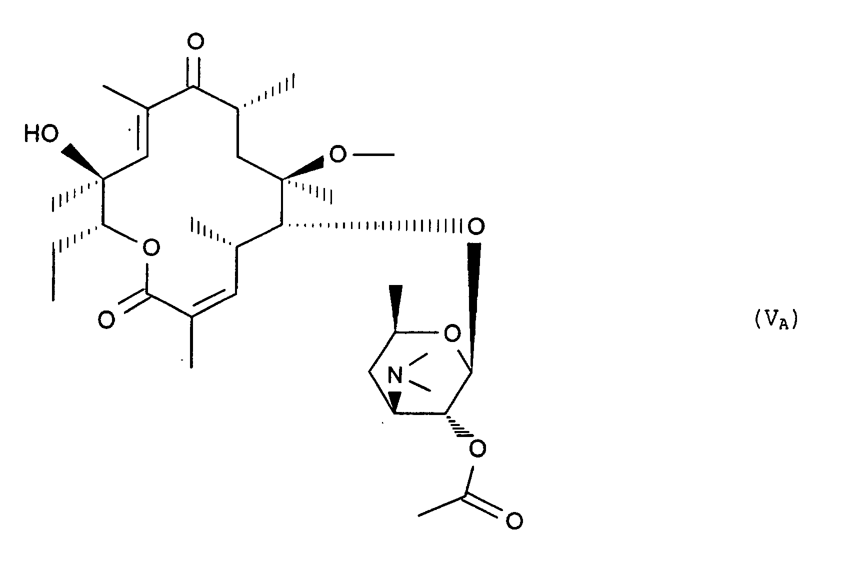 which is subjected to the action of carbonydiimidazole in order to obtain the compound of formula (VI): 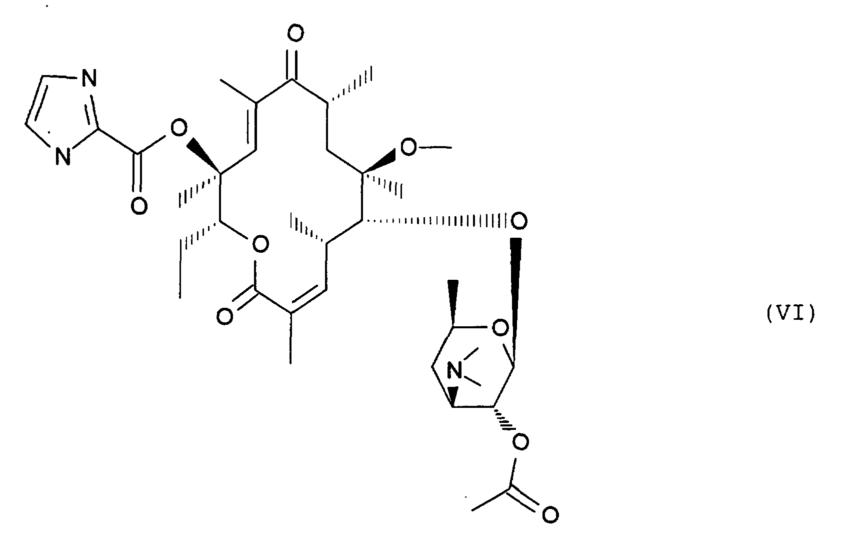 which is subjected to the action of the compound of formula (VII) :
RXNH₂ (VII)
in which R and X retain their previous meaning in order to obtain the compound of formula (I_{A}): 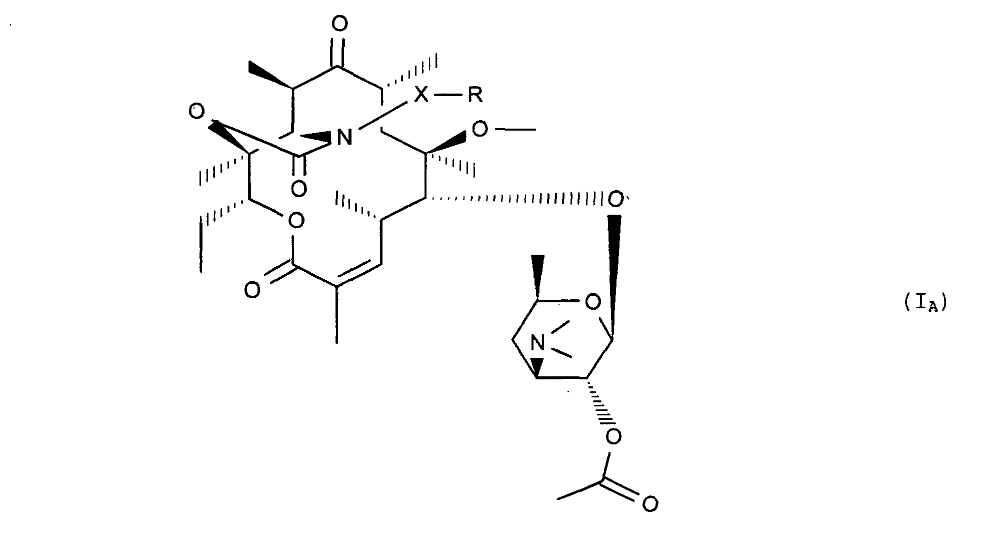 if desired, the hydroxyl in position 2' is released, in order to obtain the compound of formula (I_{B}): 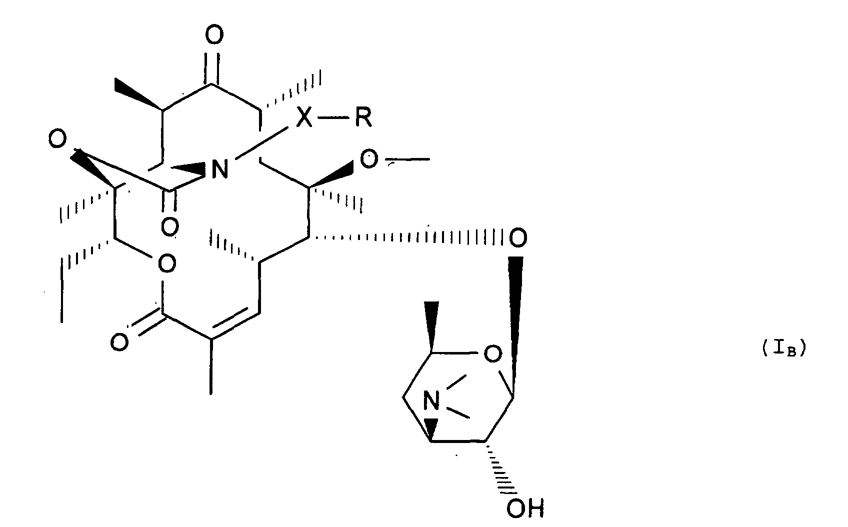 which is subjected, if desired, to the action of an esterification agent of the hydroxyl in position 2', and/or to the action of a reducing agent of the double bond in position 2(3) and/or to the action of an acid in order to form the salt.

15. The compounds of formulae (IV), (V) and (VI) defined in claim 13 or 14.

16. The following chemical products defined in claim 15:
- 3-O-de(2,6-dideoxy-3-C-methyl-3-O-methyl-alpha-L-ribohexopyranosyl)-6-O-methyl-erythromycin 11,12- carbonate cyclic 2'-acetate 3-(methanesulphonate),
- 11-deoxy-3-de[(2,6-dideoxy-3-C-methyl-3-O-methyl-alpha-L-ribo-hexopyranosyl)oxy]-6-O-methyl-2,3,10,11-tetradehydroerythromycin 2'-acetate.

## Patentansprüche

1. Verbindungen der Formel (I): 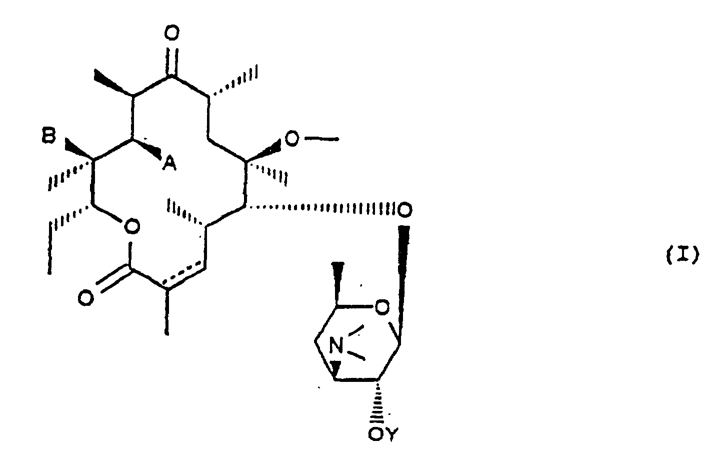 worin
- entweder A und B eine HO-Gruppe darstellen,
- oder B eine HO-Gruppe darstellt und A mit dem Kohlenstoff, der es trägt, und dem Kohlenstoff in Position 10 eine Doppelbindung bildet,
- oder A und B zusammen eine Carbonat-Gruppe bilden,
- oder A und B zusammen mit den Kohlenstoffatomen, die sie tragen, einen Ring bilden: 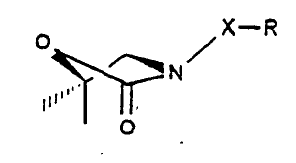
worin X eine Gruppierung CH₂, NH oder SO₂ darstellt; R eine Gruppe (CH₂)ₙAr, N-(CH₂)ₙAr oder N=CH(CH₂)ₙAr darstellt, in der n eine ganze Zahl zwischen 1 und 6 darstellt und Ar eine gegebenenfalls substituierte Aryl- oder Heteroaryl-Gruppe darstellt;
die gestrichelte Linie gegebenenfalls eine Doppelbindung bei 2(3) darstellt und Y ein Wasserstoffatom oder den Rest einer organischen Carbonsäure, der bis zu 18 Kohlenstoffatome umfaßt, darstellt;
und ihre Säureadditionssalze.

2. Verbindungen der Formel (I) gemäß Anspruch 1, die der Formel (I) entsprechen, wobei X eine Gruppierung CH₂ oder NH darstellt, R eine Gruppe (CH₂)ₙAr, N-(CH₂)ₙAr oder N=CH(CH₂)ₙAr darstellt, worin n eine ganze Zahl zwischen 1 und 6 darstellt und Ar eine gegebenenfalls substituierte Aryl- oder Heteroaryl-Gruppe darstellt; die gestrichelte Linie gegebenenfalls eine Doppelbindung bei 2(3) darstellt und Y ein Wasserstoffatom oder einen Rest einer organischen Carbonsäure, der bis zu 18 Kohlenstoffatome umfaßt, darstellt, und ihre Säureadditionssalze.

3. Verbindungen der Formel (I) nach Anspruch 1 oder Anspruch 2, wobei die gestrichelte Linie eine Doppelbindung bei 2(3) darstellt.

4. Verbindungen der Formel (I) nach Anspruch 1, 2 oder 3, wobei Y ein Wasserstoffatom darstellt.

5. Verbindungen der Formel (I) nach einem der Ansprüche 2 bis 4, wobei R eine Gruppierung (CH₂)ₙAr darstellt, n und Ar dieselbe Bedeutung wie in Anspruch 1 behalten.

6. Verbindungen der Formel (I) nach einem der Ansprüche 2 bis 5, wobei R eine Gruppe (CH₂)₃Ar, (CH₂)₄Ar oder (CH₂)₅Ar darstellt.

7. Verbindungen der Formel (I) nach einem der Ansprüche 2 bis 6, wobei Ar eine gegebenenfalls substituierte Gruppe: 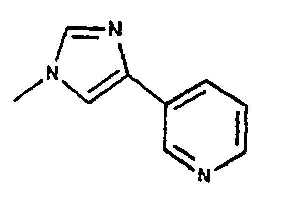 oder eine gegebenenfalls substituierte Gruppe: 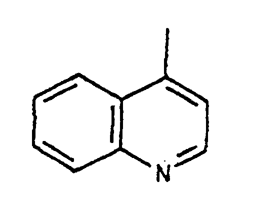 darstellt.

8. Verbindungen der Formel (I) nach Anspruch 7, wobei Ar eine Gruppe: 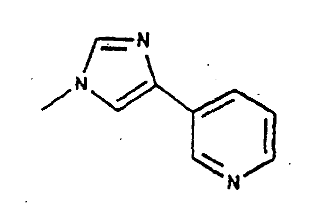 darstellt.

9. Verbindungen der Formel (I) nach Anspruch 1 mit dem Namen:
2,3-Didehydro-11,12-didesoxy-3-de[(2,6-didesoxy-3-C-methyl-3-O-methyl-α-L-ribo-hexopyranosyl)oxy]-6-O-methyl-12,11-[oxycarbonyl[[4-[4-(3-pyridinyl)-1H-imidazol-1-yl]butyl]imino]]erythromycin.

10. Verbindungen der Formel (I) nach einem der Ansprüche 1 bis 8 sowie ihre Additionssalze mit pharmazeutisch annehmbaren Säuren als Arzneimittel.

11. Verbindung nach Anspruch 9 sowie ihre Additionssalze mit pharmazeutisch annehmbaren Säuren als Arzneimittel.

12. Pharmazeutische Zusammensetzungen, die als Wirksubstanz mindestens ein Medikament nach Anspruch 10 oder 11 umfassen.

13. Verfahren zur Herstellung der Verbindungen der Formel (I) nach Anspruch 1, **dadurch gekennzeichnet, daß** man eine Verbindung der Formel (II): 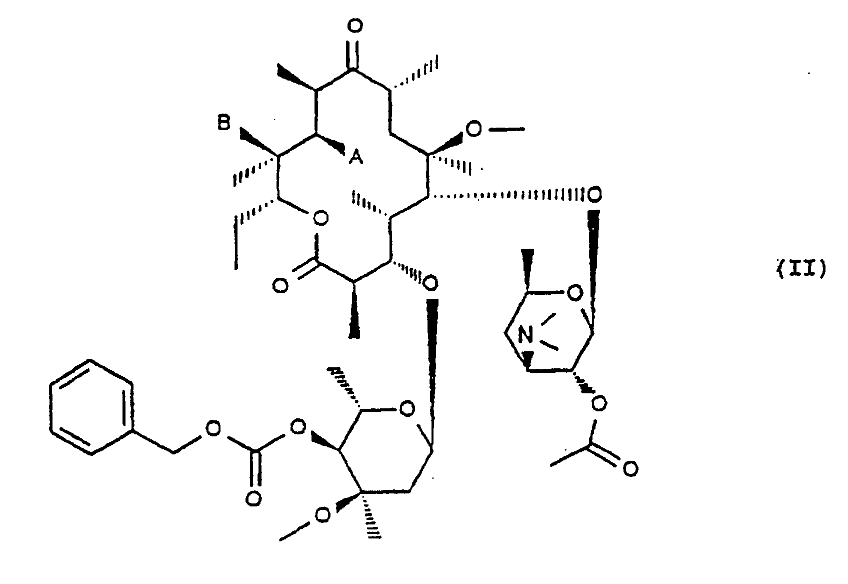 in der A und B ihre vorstehend beschriebene Bedeutung behalten, der Wirkung eines Spaltungsagenzes für die Glycosid-Bindung unterwirft, um das Hydroxyl in Position 3 freizusetzen und eine Verbindung der Formel (III) zu erhalten: 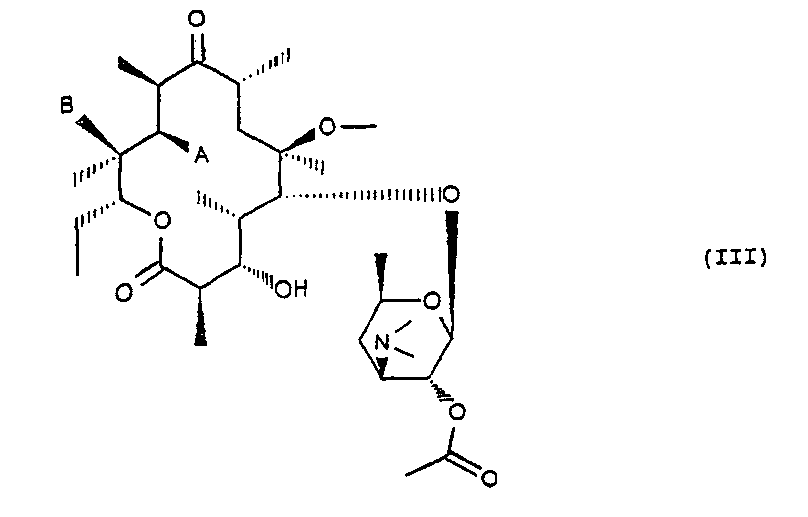 man das Hydroxyl in Position 3 als Mesylat blockiert, um die Verbindung der Formel (IV) zu erhalten: 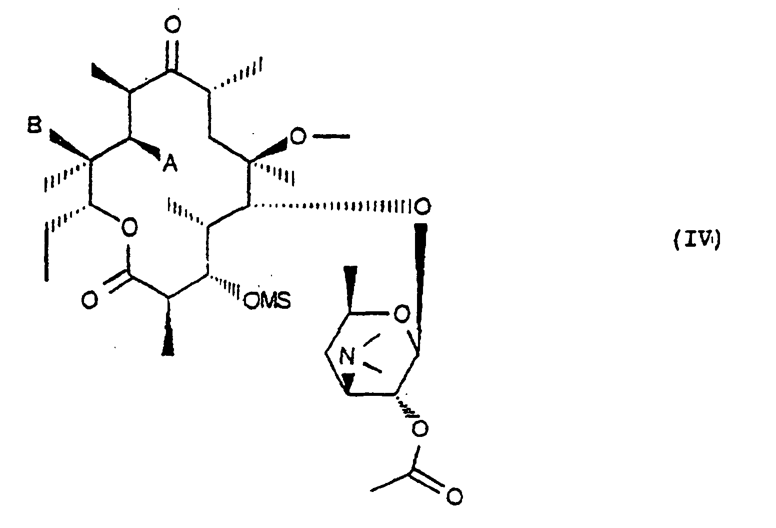 man diese der Wirkung einer Base unterwirft, um die Verbindung der Formel (V) zu erhalten: 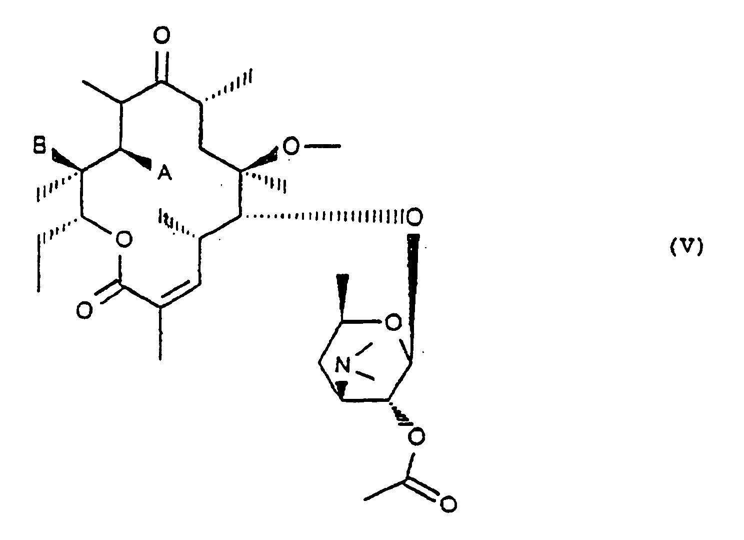 man diese, wenn gewünscht, der Wirkung eines Spaltungsagenzes für das Hydroxyl in Position 2' unterwirft, um die entsprechende Verbindung der Formel (I) zu erhalten.

14. Verfahren zur Herstellung der Verbindungen der Formel (I) nach Anspruch 1, **dadurch gekennzeichnet, daß** man eine Verbindung der Formel (II_{A}): 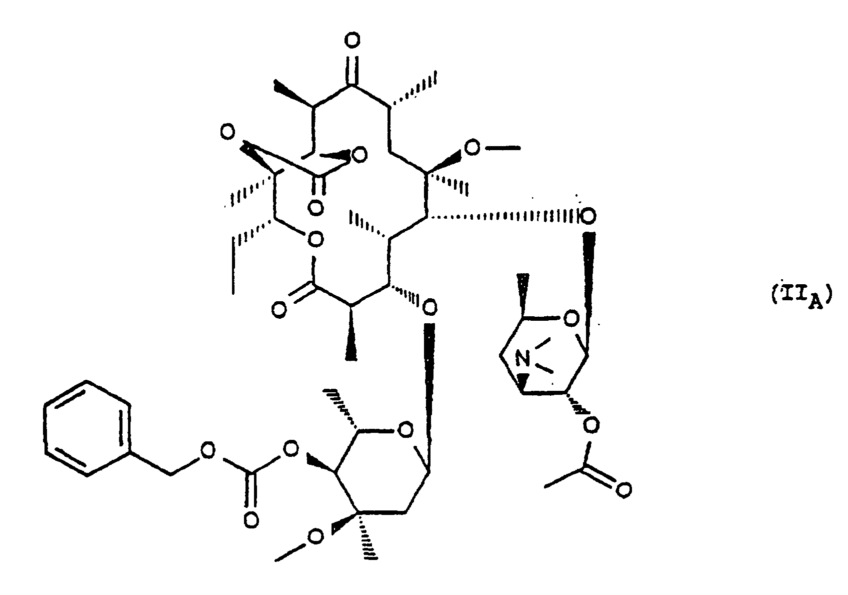 der Wirkung eines Spaltungsagenzes für die Glycosid-Bindung unterwirft, um das Hydroxyl in Position 3 freizusetzen und eine Verbindung der Formel (III_{A}) zu erhalten: 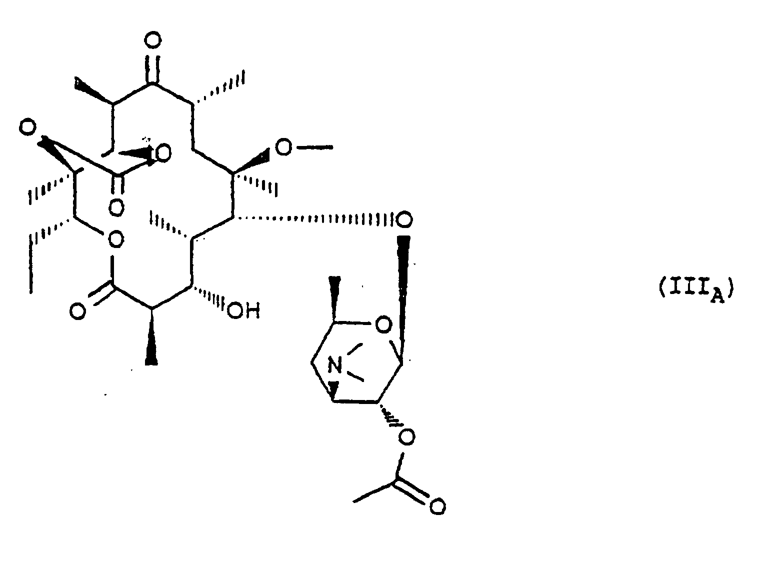 man das Hydroxyl in Position 3 als Mesylat blockiert, um die Verbindung der Formel (IV_{A}) zu erhalten: 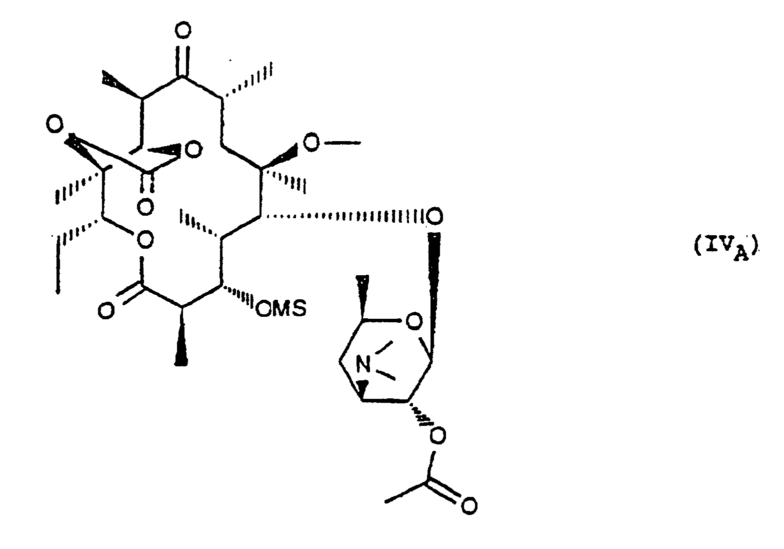 man diese der Wirkung einer Base unterwirft, um die Verbindung der Formel (VA) zu erhalten: 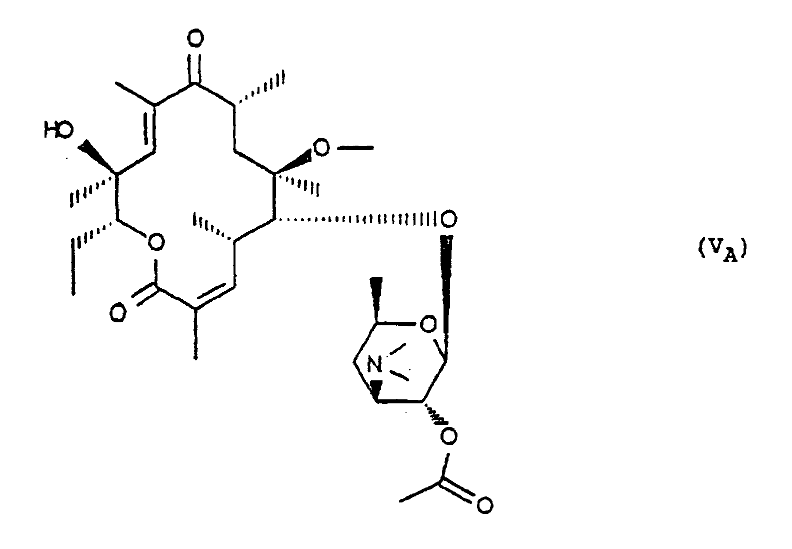 man diese der Wirkung von Carbonyldiimidazol unterwirft, um die Verbindung der Formel (VI) zu erhalten: 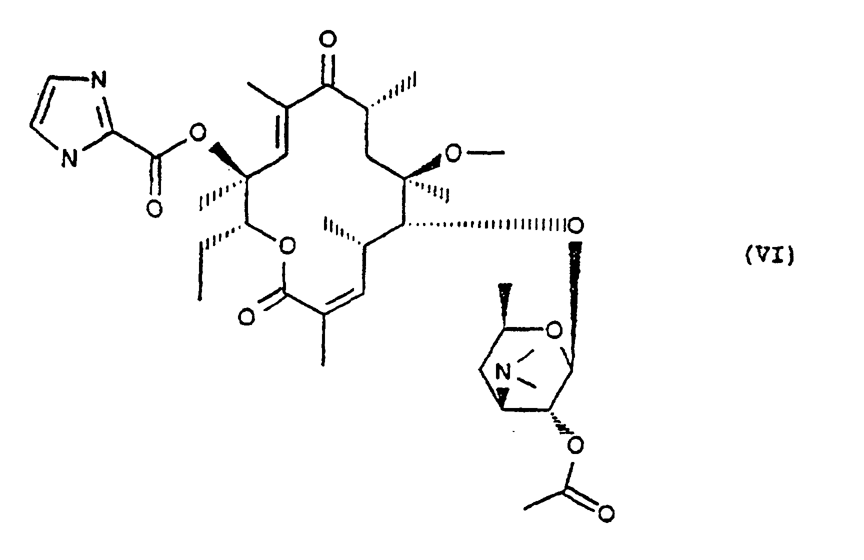 die man der Wirkung der Verbindung Formel (VII) unterwirft:
RXNH₂ (VII)
in der R und X ihre vorstehend genannte Bedeutung behalten, wobei die Verbindung der Formel (I_{A}) erhalten wird: 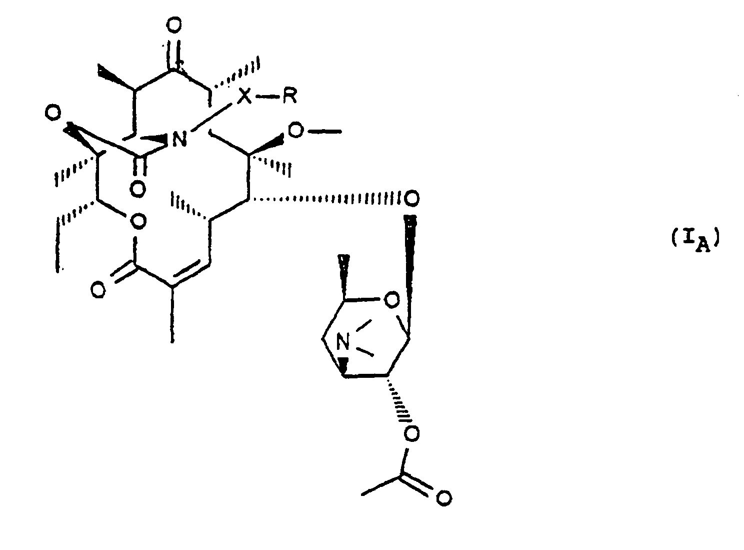 man, wenn gewünscht, das Hydroxyl in Position 2' freisetzt, um die Verbindung der Formel (I_{B}) zu erhalten: 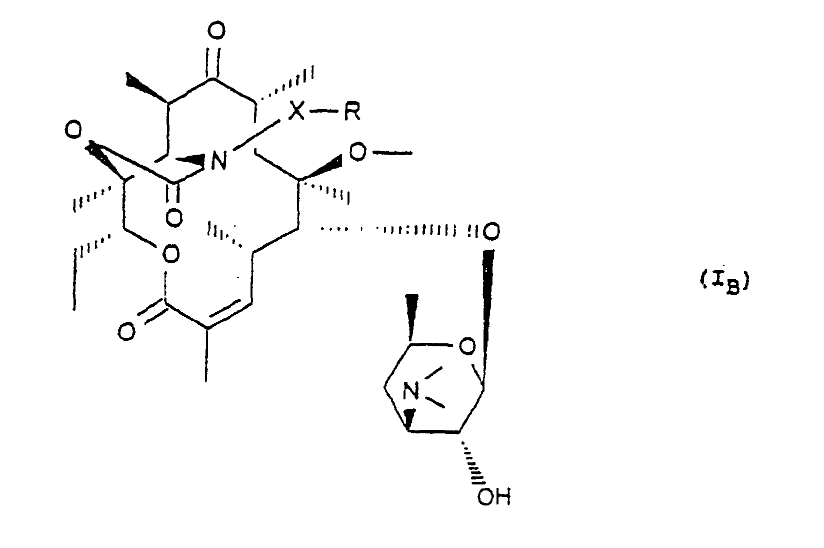 man diese, wenn gewünscht, der Wirkung eines Veresterungsmittels für das Hydroxyl in 2'-Position und/oder der Wirkung eines Reduzierungsmittels für die Doppelbindung bei 2(3) und/oder der Wirkung einer Säure zur Bildung des Salzes daraus unterwirft.

15. Verbindungen der Formeln (IV), (V) und (VI) nach Anspruch 13 oder 14.

16. Die folgenden chemischen Produkte nach Anspruch 15:
11,12-cyclisches Carbonat, 2'-Acetat, 3-(Methansulfonat) von 3-O-De(2,6-didesoxy-3-C-methyl-3-O-methyl-α-L-ribohexopyranosyl)-6-O-methylerythromycin,
2'-Acetat von 11-Desoxy-3-de[(2,6-didesoxy-3-C-methyl-3-O-methyl-α-L-ribohexopyranosyl)oxy]-6-O-methyl-2,3,10,11-tetradehydro-erythromycin.
